# EUROPEAN PATENT APPLICATION

(11) **EP 1 624 066 A2**
(43) Date of publication of application: **08.02.2006**
(21) Application number: 05105986.3
(22) Date of filing: 09.04.1998
(51) Int. Cl.: C12N 15/82

(54) **Plant fatty acid synthases and use in improved methods for production of medium-chain fatty acids**

(30) Priority: 11.04.1997 US 41815 P
(62) Divisional of application: 98915423.2
(71) Applicant: Calgene LLC, Davis, California 95616 (US)
(72) Inventor: Dehesh, Katayoon, Vacaville,CA 95687 (US)
(74) Representative: Helbing, Jörg

(57) **Abstract**

By this invention, compositions and methods of use related to β-ketoacyl-ACP synthase of special interest are synthases obtainable from *Cuphea* species. Amino acid and nucleic acid for synthase protein factors are provided, as well as methods to utilize such sequences in constructs for production of genetically engineered plants having altered fatty acid compositions. Of particular interest is the expression of synthase protein factors in conjunction with expression of plant medium-chain acyl-ACP thioesterases for production of increased levels and/or modified ratios of medium-chain fatty acids in oils of transgenic plant seeds.

### Annex to the application documents - subsequently filed sequences listing

SEQUENCE LISTING

<110> Calgene LLC

<120> Plant Fatty Acid Synthases and Use in Improved Methods for Production of
Medium-Chain Fatty Acids

<130> 051364ep/JH/ml
<140> EP 05105986.3

<160> 17

<170> PatentIn version 3.1

<210> 1
<211> 1348
<212> DNA
<213> Cuphea hookeriana

<400> 1
agctccaccg cggtggcggc cgctctagaa ctagtggatc ccccgggctg caggaattcg 60

gcacgagccg atctcggtgc cgaccgcctc tccaagatcg acaaggagag agccggagtg 120

ctggtcggaa caggaatggg tggtctgact gtcttctctg acggggttca gtctcttatc 180

gagaagggtc accggaaaat cacccctttc ttcatcccct atgccattac aaacatgggg 240

tctgccctgc tcgctatcga atttggtctc atgggcccaa actattcaat ttccactgca 300

tgtgccactt ccaactactg cttccatgct gccgctaatc atatccgccg tggtgaggct 360

gatcttatga ttgctggagg cactgaggcc gcaatcattc caattgggtt gggaggcttt 420

gtggcttgca gggctttgtc tcaaaggaac gatgacccgc agactgcctc taggccctgg 480

gataaagacc gtgatggttt tgtgatgggt gaaggtgctg gagtgttggt gatggagagc 540

ttggaacatg caatgagacg aggagcaccg attattgcag agtatttggg aggtgcaatc 600

aactgtgatg cttatcacat gactgatcca agggctgatg gtcttggtgt ctcttcttgc 660

attgagagta gccttgaaga tgctggcgtc tcacctgaag aggtcaatta cataaatgct 720

catgcgactt ctactctagc tggggatctc gccgagataa atgccatcaa gaaggttttc 780

aagaacacaa aggatatcaa aattaatgca actaagtcaa tgatcggaca ctgtcttgga 840

gcatctggag gtcttgaagc tatagcgact attaagggaa taaacaccgg ctggcttcat 900

cccagcatta atcaattcaa tcctgagcca tcggtggagt tcgacactgt tgccaacaag 960

aagcagcaac acgaagttaa cgttgcgatc tcgaattcat tcggatttgg aggccacaac 1020

tcagtcgtgg ctttctcggc tttcaagcca tgattaccca tttcacaagg tacttgtcat 1080

tgagaatacg gattatggac ttgcagagta atttccccat gtttgtcgga agagcatatt 1140

accacggttg tccgtcaaac ccatttagga tactgttcta tgtaataaaa ctaaggatta 1200

ttaatttccc ttttaatcct gtctccagtt tgagcatgaa attatattta ttttatctta 1260

gaaaggtcaa ataagatttt gttttacctc tgtaaaactt ttgtttgtat tggaaaggaa 1320

gtgccgtctc aaaaaaaaaa aaaaaaaa                                    1348

<210>  2
<211>  350
<212>  PRT
<213>  Cuphea hookeriana

<400>  2

Ser Ser Thr Ala Val Ala Ala Ala Leu Glu Leu Val Asp Pro Pro Gly
1               5                   10                  15

Cys Arg Asn Ser Ala Arg Ala Asp Leu Gly Ala Asp Arg Leu Ser Lys
            20                  25                  30

Ile Asp Lys Glu Arg Ala Gly Val Leu Val Gly Thr Gly Met Gly Gly
        35                  40                  45

Leu Thr Val Phe Ser Asp Gly Val Gln Ser Leu Ile Glu Lys Gly His
    50                  55                  60

Arg Lys Ile Thr Pro Phe Phe Ile Pro Tyr Ala Ile Thr Asn Met Gly
65                  70                  75                  80

Ser Ala Leu Leu Ala Ile Glu Phe Gly Leu Met Gly Pro Asn Tyr Ser
                85                  90                  95

Ile Ser Thr Ala Cys Ala Thr Ser Asn Tyr Cys Phe His Ala Ala Ala
            100                 105                 110

Asn His Ile Arg Arg Gly Glu Ala Asp Leu Met Ile Ala Gly Gly Thr
        115                 120                 125

Glu Ala Ala Ile Ile Pro Ile Gly Leu Gly Gly Phe Val Ala Cys Arg
    130                 135                 140

Ala Leu Ser Gln Arg Asn Asp Asp Pro Gln Thr Ala Ser Arg Pro Trp
145                 150                 155                 160

Asp Lys Asp Arg Asp Gly Phe Val Met Gly Glu Gly Ala Gly Val Leu
                165                 170                 175

Val Met Glu Ser Leu Glu His Ala Met Arg Arg Gly Ala Pro Ile Ile
            180                 185                 190

Ala Glu Tyr Leu Gly Gly Ala Ile Asn Cys Asp Ala Tyr His Met Thr
        195                 200                 205

Asp Pro Arg Ala Asp Gly Leu Gly Val Ser Ser Cys Ile Glu Ser Ser
    210                 215                 220

Leu Glu Asp Ala Gly Val Ser Pro Glu Glu Val Asn Tyr Ile Asn Ala
225                 230                 235                 240

His Ala Thr Ser Thr Leu Ala Gly Asp Leu Ala Glu Ile Asn Ala Ile
                245                 250                 255

Lys Lys Val Phe Lys Asn Thr Lys Asp Ile Lys Ile Asn Ala Thr Lys
            260                 265                 270

Ser Met Ile Gly His Cys Leu Gly Ala Ser Gly Gly Leu Glu Ala Ile
        275                 280                 285

Ala Thr Ile Lys Gly Ile Asn Thr Gly Trp Leu His Pro Ser Ile Asn
    290                 295                 300

Gln Phe Asn Pro Glu Pro Ser Val Glu Phe Asp Thr Val Ala Asn Lys
305                 310                 315                 320

Lys Gln Gln His Glu Val Asn Val Ala Ile Ser Asn Ser Phe Gly Phe
                325                 330                 335

Gly Gly His Asn Ser Val Val Ala Phe Ser Ala Phe Lys Pro
            340                 345                 350

<210>  3
<211>  1703
<212>  DNA
<213>  Cuphea hookeriana

<220>
<221>  misc_feature
<222>  (44)..(1499)
<223>  unsure at all n locations

<400>  3
aaattaaccc tcactaaagg gaacaaaagc tggagctcca ccgnggtggc ggccgctcta      60

gaactagtgg atcccccggg ctgcaggaat tcggcacgag ccggcatggg cctcgtctcc     120

gtattcggct ccgacgtcga ctcttattac gaaaagctcc tctccggcga gagcgggatc     180

agcttaatcg accgcttcga cgcttccaag ttccccacca ggttcggcgg ccagatccgg     240

ggattcaacg cgacgggata catcgacggg aagaacgaca ggaggctcga cgattgcctc     300

gctactgcat tgtcgccggg aagaaggctc tcgaaaattc cgatctcggc ggtgaaagcc     360

tctccaagat tgataaggag agagctggag tgctagttgg aactggtatg ggtggcctaa     420

ccgtcttctc tgacggggtt cagaatctca tcgagaaagg tcaccggaag atctccccgt 480
ttttcattcc ctatgccatt acaaacatgg ggtctgctct gcttgccatc gatttgggtc 540
tgatgggccc aaactattcg atttcaactg catgtgctac ttccaactac tgcttttatg 600
ccgctgccaa tcatatccgc cgaggcgagg ctgacctcat gattgctgga ggaactgagg 660
ctgcaatcat tccaattggg ttaggaggat tcgttgcctg cagggcttta tctcaaagga 720
atgatgaccc tcagactgcc tcaaggccgt gggataagga ccgtgatggt tttgtgatgg 780
gcgaaggggc tggagtattg gttatggaga gcttggaaca tgcaatgaaa cgaggagcgc 840
cgattattgc agaatatttg ggaggtgcag tcaattgtga tgcttatcat atgactgatc 900
caagggctga tgggcttggt gtctcctctt gcattgagag cagtctggaa gatgctgggg 960
tctcacctga agaggtcaat tacataaatg ctcatgcgac ttccactctt gctggggatc 1020
ttgccgagat aaatgccatc aagaaggttt tcaagaacac caaggaaatc acaatcaatg 1080
caactaagtc gatgatcgga cactgtcttg gagcatcagg gggtcttgaa gccattgcga 1140
caattaaggg aataaccacc ggctggcttc atcccagcat aaaccaattc aatcccgagc 1200
catcagtgga attcgacaca gttgccaaca agaagcagca acatgaagtg aatgttgcta 1260
tctcaaattc attcggattc ggaggccaca actcagttgt agctttctca gccttcaagc 1320
catgattact cggttcaaat gcaaatttgt tgctgagaca gtgagcttca acttgcagag 1380
caatttttta catgccttgt cgtcggaaga gcgtaatacc gggatagttc cttgatagtt 1440
catttaggat gttttactgc aataatcgaa gattatttcc attctaatcc agtctccgnc 1500
gagtttgaga atctatctgt ttgtattaga aagaacgagg caagattttg tttcatgttt 1560
gtgtttgtat tactttcttt ttgcccttgt caatggcatt taagataagc ttataaaaaa 1620
aaaaaaaaaa aaaaaaactc gagggggggc ccggtaccca attcgcccta tagtgagtcg 1680
tatgacaatt cactgtccgt cgg 1703

<210> 4
<211> 441
<212> PRT
<213> Cuphea hookeriana

<220>
<221> Unsure
<222> (15)..(15)
<223> X = Any Amino Acid

<220>
<221> misc_feature
<222> (15)..(15)
<223> X = Any Amino Acid

<400> 4

Lys Leu Thr Leu Thr Lys Gly Asn Lys Ser Trp Ser Ser Thr Xaa Val
1               5                   10                  15

Ala Ala Ala Leu Glu Leu Val Asp Pro Pro Gly Cys Arg Asn Ser Ala
            20                  25                  30

Arg Ala Gly Met Gly Leu Val Ser Val Phe Gly Ser Asp Val Asp Ser
        35                  40                  45

Tyr Tyr Glu Lys Leu Leu Ser Gly Glu Ser Gly Ile Ser Leu Ile Asp
    50                  55                  60

Arg Phe Asp Ala Ser Lys Phe Pro Thr Arg Phe Gly Gly Gln Ile Arg
65                  70                  75                  80

Gly Phe Asn Ala Thr Gly Tyr Ile Asp Gly Lys Asn Asp Arg Arg Leu
                85                  90                  95

Asp Asp Cys Leu Arg Tyr Cys Ile Val Ala Gly Lys Lys Ala Leu Glu
            100                 105                 110

Asn Ser Asp Leu Gly Gly Glu Ser Leu Ser Lys Ile Asp Lys Glu Arg
        115                 120                 125

Ala Gly Val Leu Val Gly Thr Gly Met Gly Gly Leu Thr Val Phe Ser
    130                 135                 140

Asp Gly Val Gln Asn Leu Ile Glu Lys Gly His Arg Lys Ile Ser Pro
145                 150                 155                 160

Phe Phe Ile Pro Tyr Ala Ile Thr Asn Met Gly Ser Ala Leu Leu Ala
                165                 170                 175

Ile Asp Leu Gly Leu Met Gly Pro Asn Tyr Ser Ile Ser Thr Ala Cys
            180                 185                 190

Ala Thr Ser Asn Tyr Cys Phe Tyr Ala Ala Ala Asn His Ile Arg Arg
        195                 200                 205

Gly Glu Ala Asp Leu Met Ile Ala Gly Gly Thr Glu Ala Ala Ile Ile
    210                 215                 220

Pro Ile Gly Leu Gly Gly Phe Val Ala Cys Arg Ala Leu Ser Gln Arg
225                 230                 235                 240

Asn Asp Asp Pro Gln Thr Ala Ser Arg Pro Trp Asp Lys Asp Arg Asp

245                 250                 255

Gly Phe Val Met Gly Glu Gly Ala Gly Val Leu Val Met Glu Ser Leu
            260                 265                 270

Glu His Ala Met Lys Arg Gly Ala Pro Ile Ile Ala Glu Tyr Leu Gly
        275                 280                 285

Gly Ala Val Asn Cys Asp Ala Tyr His Met Thr Asp Pro Arg Ala Asp
    290                 295                 300

Gly Leu Gly Val Ser Ser Cys Ile Glu Ser Ser Leu Glu Asp Ala Gly
305                 310                 315                 320

Val Ser Pro Glu Glu Val Asn Tyr Ile Asn Ala His Ala Thr Ser Thr
                325                 330                 335

Leu Ala Gly Asp Leu Ala Glu Ile Asn Ala Ile Lys Lys Val Phe Lys
            340                 345                 350

Asn Thr Lys Glu Ile Thr Ile Asn Ala Thr Lys Ser Met Ile Gly His
        355                 360                 365

Cys Leu Gly Ala Ser Gly Gly Leu Glu Ala Ile Ala Thr Ile Lys Gly
    370                 375                 380

Ile Thr Thr Gly Trp Leu His Pro Ser Ile Asn Gln Phe Asn Pro Glu
385                 390                 395                 400

Pro Ser Val Glu Phe Asp Thr Val Ala Asn Lys Lys Gln Gln His Glu
                405                 410                 415

Val Asn Val Ala Ile Ser Asn Ser Phe Gly Phe Gly Gly His Asn Ser
            420                 425                 430

Val Val Ala Phe Ser Ala Phe Lys Pro
        435                 440

<210>  5
<211>  2046
<212>  DNA
<213>  Cuphea hookeriana

<400>  5

actaaaggga acaaaagctg gagctccacc gcggtggcgg ccgctctaga actagtggat      60

cccccgggct gcaggaattc ggcacgagtt ttcttacttg ggtcggctca gctcaggtgt     120

tccaatggcg accgcttctt gcatggttgc gtcccctttc tgtacgtggc tcgtagctgc 180

atgcatgccc acttcatccg acaacgaccc acgttccctt tcccacaagc ggctccgcct 240

ctcccgtcgc cggaggactc tctcctccca ttgctccctc cgcggatcca ccttccaatg 300

cctcgatcct tgcaaccagc aacgcttcct cggggataac ggattcgctt ccctcttcgg 360

atccaagcct cttcgttcaa atcgcggcca cctgaggctc ggccgcactt cccattccgg 420

ggaggtcatg gctgtggcta tgcaacctgc acaggaagtc tccacaaata agaaacctgc 480

taccaagcaa aggcgagtag ttgtgacagg tatgggcgtg gtgactcctc taggccatga 540

ccccgatgtt tactacaaca atctcctaga cggaataagt ggcataagtg agatagagaa 600

cttcgactgc tctcagtttc ccacgagaat tgccggagag atcaagtctt tttccacaga 660

tggctgggtg gccccaaagt tctccgagag gatggacaag ttcatgcttt acatgctgac 720

tgcaggcaag aaagcattag cagatggtgg aatcactgaa gatgcgatga aagagctcaa 780

taaaagaaag tgtggagttc tcattggctc cggattgggc ggtatgaagg tattcagcga 840

ttccattgaa gctctgagga cttcatataa gaagatcagt cccttttgtg tacctttttc 900

taccacaaat atgggatccg ctattcttgc aatggacttg ggatggatgg gccctaacta 960

ttcgatatca actgcctgtg caacaagtaa cttctgtata ctgaatgctg cgaaccacat 1020

aatcaaaggc gaagcagaca tgatgctttg tggtggctcg gatgcggccg ttttacctgt 1080

tggtttggga ggtttcgtag catgccgagc tttgtcacag aggaataatg accctaccaa 1140

agcttcgaga ccatgggaca gtaatcgtga tggatttgtg atgggagaag gagctggagt 1200

tttacttctt gaggagttag agcatgcaaa gaaaagaggt gcaaccattt atgcggaatt 1260

tctaggtggg agtttcactt gcgacgccta ccacatgacc gagcctcacc ctgaaggagc 1320

tggtgtgatc ctctgcatag agaaggcctt ggctcagtcc ggagtctcga gggaagacgt 1380

aaattacata aatgcgcatg caacttccac tcctgctgga gatatcaagg aataccaagc 1440

tctcgcccac tgtttcggcc aaaacagtga gctgagagtg aattccacca aatcgatgat 1500

cggtcacctt cttggaggag ctggtggcgt agaagcagtt gcagtagttc aggcaataag 1560

gacaggatgg atccatccaa atattaattt ggaagacccg gacgaaggcg tggatgcaaa 1620

actgctcgtc ggccctaaga aggagaaact gaaggtcaag gtcggtttgt ccaattcatt 1680

tgggttcggc ggccataact catccatact atttgccccc tgcaactaga aaagagtctg 1740

tggaagccga gagtctttga gaactcatgc acgttagtag cttcttatgc ctctgaaacc 1800

gagatagacc ggctactcga ggggatgcca aagatactcc ttgccggtat tggtgttaag 1860

agatcactgc ttgtcccttt tattttcttc ttcttttgag agctttaacc gaggtagtcg 1920

tattttcgag cttttcgaat acatgttcgt tatcggatca atgtgtttct tctaagatca 1980

tttgtaatgc atattttgaa aaaccacatc tcagtatgca aaataaaaaa aaaaaaaaaa 2040

aaaaaa 2046

<210> 6
<211> 534
<212> PRT
<213> Cuphea hookeriana

<400> 6

Met Ala Thr Ala Ser Cys Met Val Ala Ser Pro Phe Cys Thr Trp Leu
1               5                   10                  15

Val Ala Ala Cys Met Pro Thr Ser Ser Asp Asn Asp Pro Arg Ser Leu
            20                  25                  30

Ser His Lys Arg Leu Arg Leu Ser Arg Arg Arg Arg Thr Leu Ser Ser
        35                  40                  45

His Cys Ser Leu Arg Gly Ser Thr Phe Gln Cys Leu Asp Pro Cys Asn
    50                  55                  60

Gln Gln Arg Phe Leu Gly Asp Asn Gly Phe Ala Ser Leu Phe Gly Ser
65                  70                  75                  80

Lys Pro Leu Arg Ser Asn Arg Gly His Leu Arg Leu Gly Arg Thr Ser
                85                  90                  95

His Ser Gly Glu Val Met Ala Val Ala Met Gln Pro Ala Gln Glu Val
            100                 105                 110

Ser Thr Asn Lys Lys Pro Ala Thr Lys Gln Arg Arg Val Val Val Thr
        115                 120                 125

Gly Met Gly Val Val Thr Pro Leu Gly His Asp Pro Asp Val Tyr Tyr
    130                 135                 140

Asn Asn Leu Leu Asp Gly Ile Ser Gly Ile Ser Glu Ile Glu Asn Phe
145                 150                 155                 160

Asp Cys Ser Gln Phe Pro Thr Arg Ile Ala Gly Glu Ile Lys Ser Phe
                165                 170                 175

Ser Thr Asp Gly Trp Val Ala Pro Lys Phe Ser Glu Arg Met Asp Lys
            180                 185                 190

Phe Met Leu Tyr Met Leu Thr Ala Gly Lys Lys Ala Leu Ala Asp Gly

        195                 200                 205

Gly Ile Thr Glu Asp Ala Met Lys Glu Leu Asn Lys Arg Lys Cys Gly
    210                 215                 220

Val Leu Ile Gly Ser Gly Leu Gly Gly Met Lys Val Phe Ser Asp Ser
225                 230                 235                 240

Ile Glu Ala Leu Arg Thr Ser Tyr Lys Lys Ile Ser Pro Phe Cys Val
                245                 250                 255

Pro Phe Ser Thr Thr Asn Met Gly Ser Ala Ile Leu Ala Met Asp Leu
            260                 265                 270

Gly Trp Met Gly Pro Asn Tyr Ser Ile Ser Thr Ala Cys Ala Thr Ser
        275                 280                 285

Asn Phe Cys Ile Leu Asn Ala Ala Asn His Ile Ile Lys Gly Glu Ala
    290                 295                 300

Asp Met Met Leu Cys Gly Gly Ser Asp Ala Ala Val Leu Pro Val Gly
305                 310                 315                 320

Leu Gly Gly Phe Val Ala Cys Arg Ala Leu Ser Gln Arg Asn Asn Asp
                325                 330                 335

Pro Thr Lys Ala Ser Arg Pro Trp Asp Ser Asn Arg Asp Gly Phe Val
            340                 345                 350

Met Gly Glu Gly Ala Gly Val Leu Leu Leu Glu Glu Leu Glu His Ala
        355                 360                 365

Lys Lys Arg Gly Ala Thr Ile Tyr Ala Glu Phe Leu Gly Gly Ser Phe
    370                 375                 380

Thr Cys Asp Ala Tyr His Met Thr Glu Pro His Pro Glu Gly Ala Gly
385                 390                 395                 400

Val Ile Leu Cys Ile Glu Lys Ala Leu Ala Gln Ser Gly Val Ser Arg
                405                 410                 415

Glu Asp Val Asn Tyr Ile Asn Ala His Ala Thr Ser Thr Pro Ala Gly
            420                 425                 430

Asp Ile Lys Glu Tyr Gln Ala Leu Ala His Cys Phe Gly Gln Asn Ser
        435                 440                 445

Glu Leu Arg Val Asn Ser Thr Lys Ser Met Ile Gly His Leu Leu Gly
    450                 455                 460

Gly Ala Gly Gly Val Glu Ala Val Ala Val Val Gln Ala Ile Arg Thr
465                 470                 475                 480

Gly Trp Ile His Pro Asn Ile Asn Leu Glu Asp Pro Asp Glu Gly Val
                485                 490                 495

Asp Ala Lys Leu Leu Val Gly Pro Lys Lys Glu Lys Leu Lys Val Lys
            500                 505                 510

Val Gly Leu Ser Asn Ser Phe Gly Phe Gly Gly His Asn Ser Ser Ile
        515                 520                 525

Leu Phe Ala Pro Cys Asn
    530

<210>  7
<211>  1921
<212>  DNA
<213>  Cuphea hookeriana

<400>  7
cggcacgagg tcacctctta cctcgcctgc ttcgagccct gccatgacta ctacacctcc      60

gcatccttgt tcggatccag gcccatccgc accacccgca ggcaccggag gctcaatcga     120

gcttcccctt ccggggaggc aatggctgtg gctctgcaac ctgcacagga agttaccaca     180

aagaagaagc caagtatcaa acagcggcga gtagttgtga ctggaatggg tgtggtgact     240

cctctaggcc atgaccctga tgttttctac aataatctgc ttgatggaac gagtggcata     300

agtgagatag agacctttga ttgtgctcaa tttcctacga gaattgctgg agagatcaag     360

tctttctcca cagatggttg ggtggccccg aagctctcca agaggatgga caagttcatg     420

ctttacatgc tgactgccgg caagaaagca ttaacaaatg gtggaatcac cgaagatgtg     480

atgaaagagc tagataaaag aaaatgcgga gttctcattg gctcagcaat gggtggaatg     540

aaggtattca atgatgccat tgaagcccta aggatttcat ataagaagat gaatcccttt     600

tgtgtacctt tcgctaccac aaatatggga tcagctatgc ttgcaatgga cttgggatgg     660

atgggcccca actactcgat atctactgct tgtgcaacga gtaacttttg tatcctgaat     720

gctgcgaacc acataatcag aggcgaagca gatgtgatgc tttgcggggg ctcagatgcg     780

gtaatcatac ctattggtat gggaggtttt gttgcatgcc gagctttgtc acagagaaat     840

gccgacccta ctaaagcttc aagaccatgg gacagtaatc gtgatggatt tgttatgggg     900

gaaggagctg gagtgctact actagaggag ttagagcatg caaagaaaag aggtgcgact     960

atttacgcag aatttctagg tggaagtttc acttgcgatg cctaccacat gaccgagcct    1020

caccctgatg gagctggagt gattctctgc atagagaagg ctttggctca gtcaggagtc 1080

tctagggaag acgtaaatta cataaatgca catgccacat ccactccagc tggagatatc 1140

aaagagtacc aagctcttat ccactgtttc ggccaaaaca acgagttaaa agtgaattct 1200

accaaatcaa tgattggtca ccttctcgga gcagccggtg gtgtggaagc agtttcagta 1260

gttcaggcaa taaggactgg gtggatccat ccgaatatta atttggaaaa cccagatgaa 1320

ggcgtggata ccaaattgct cgtgggccct aagaaggaga gactgaacat taaggtcggt 1380

ttgtctaatt cattcgggtt tggtgggcac aactcgtcca tactcttcgc cccttacaac 1440

tagggcgttt catgtgtgga attctactca atctatcaaa gctgaagttt tgaggactcc 1500

agcatgttgg tagctcctta cgtctctaga catgcccatg agttttgtgt cgggagctgt 1560

agtcggaacc atgacggatt gagtactcat ggcgacacag gatatactcc ttgctagaat 1620

tgttagagca ctattcatta tcccattttt tttctgaaat ctccctcctt acggtagttg 1680

tactttcgag cgtttcatcg agtcagtgaa gaagagaaca aagctaactc gggcacgtag 1740

taaccatttg ccctttgttt tgctctctat tttatcgccg ttttgtgggt taaaatttgt 1800

aaaactagac gactggtttg ttttctcttg atcattggag atgtatggcc atatttgcct 1860

ttcattgatg ataaaaaaaa aaaaaaaaaa aaaaaaaaaa aaaaaaaaaa aaaaaaaaaa 1920

a 1921

<210> 8
<211> 420
<212> PRT
<213> Cuphea hookeriana

<400> 8

Lys Lys Lys Pro Ser Ile Lys Gln Arg Arg Val Val Val Thr Gly Met
1               5                   10                  15

Gly Val Val Thr Pro Leu Gly His Asp Pro Asp Val Phe Tyr Asn Asn
            20                  25                  30

Leu Leu Asp Gly Thr Ser Gly Ile Ser Glu Ile Glu Thr Phe Asp Cys
        35                  40                  45

Ala Gln Phe Pro Thr Arg Ile Ala Gly Glu Ile Lys Ser Phe Ser Thr
    50                  55                  60

Asp Gly Trp Val Ala Pro Lys Leu Ser Lys Arg Met Asp Lys Phe Met
65                  70                  75                  80

Leu Tyr Met Leu Thr Ala Gly Lys Lys Ala Leu Thr Asn Gly Gly Ile
                85                  90                  95

Thr Glu Asp Val Met Lys Glu Leu Asp Lys Arg Lys Cys Gly Val Leu
            100                 105                 110

Ile Gly Ser Ala Met Gly Gly Met Lys Val Phe Asn Asp Ala Ile Glu
        115                 120                 125

Ala Leu Arg Ile Ser Tyr Lys Lys Met Asn Pro Phe Cys Val Pro Phe
    130                 135                 140

Ala Thr Thr Asn Met Gly Ser Ala Met Leu Ala Met Asp Leu Gly Trp
145                 150                 155                 160

Met Gly Pro Asn Tyr Ser Ile Ser Thr Ala Cys Ala Thr Ser Asn Phe
                165                 170                 175

Cys Ile Leu Asn Ala Ala Asn His Ile Ile Arg Gly Glu Ala Asp Val
            180                 185                 190

Met Leu Cys Gly Gly Ser Asp Ala Val Ile Ile Pro Ile Gly Met Gly
        195                 200                 205

Gly Phe Val Ala Cys Arg Ala Leu Ser Gln Arg Asn Ala Asp Pro Thr
    210                 215                 220

Lys Ala Ser Arg Pro Trp Asp Ser Asn Arg Asp Gly Phe Val Met Gly
225                 230                 235                 240

Glu Gly Ala Gly Val Leu Leu Leu Glu Glu Leu Glu His Ala Lys Lys
                245                 250                 255

Arg Gly Ala Thr Ile Tyr Ala Glu Phe Leu Gly Gly Ser Phe Thr Cys
            260                 265                 270

Asp Ala Tyr His Met Thr Glu Pro His Pro Asp Gly Ala Gly Val Ile
        275                 280                 285

Leu Cys Ile Glu Lys Ala Leu Ala Gln Ser Gly Val Ser Arg Glu Asp
    290                 295                 300

Val Asn Tyr Ile Asn Ala His Ala Thr Ser Thr Pro Ala Gly Asp Ile
305                 310                 315                 320

Lys Glu Tyr Gln Ala Leu Ile His Cys Phe Gly Gln Asn Asn Glu Leu
                325                 330                 335

Lys Val Asn Ser Thr Lys Ser Met Ile Gly His Leu Leu Gly Ala Ala

            340                 345                 350

Gly Gly Val Glu Ala Val Ser Val Val Gln Ala Ile Arg Thr Gly Trp
        355                 360                 365

Ile His Pro Asn Ile Asn Leu Glu Asn Pro Asp Glu Gly Val Asp Thr
    370                 375                 380

Lys Leu Leu Val Gly Pro Lys Lys Glu Arg Leu Asn Ile Lys Val Gly
385                 390                 395                 400

Leu Ser Asn Ser Phe Gly Phe Gly Gly His Asn Ser Ser Ile Leu Phe
                405                 410                 415

Ala Pro Tyr Asn
            420

<210> 9
<211> 1713
<212> DNA
<213> Cuphea pulcherrima

<400> 9
ctggtacgcc tgcaggtacc ggtccggaat tcccgggtcg acccacgcgt ccgtcttccc 60

actccgatcg ttcttcttcc accgcatctc ttctcttctc ttggcttctc cgccatcctc 120

cgccgccatg cattccctcc agtcaccctc ccttcgggcc tccccgctcg accccttccg 180

ccccaaatca tccaccgtcc gccccctcca ccgagcatca attcccaacg tccgggccgc 240

ttcccccacc gtctccgctc ccaagcgcga gaccgacccc aagaagcgcg tcgtgatcac 300

cggaatgggc cttgtctccg ttttcggctc cgacgtcgat gcgtactacg acaagctcct 360

gtcaggcgag agcgggatcg gcccaatcga ccgcttcgac gcctccaagt tccccaccag 420

gttcggcggc cagattcgtg gcttcaactc catgggatac attgacggca aaaacgacag 480

gcggcttgat gattgccttc gctactgcat tgtcgccggg aagaagtctc ttgaggacgc 540

cgatctcggt gccgaccgcc tctccaagat cgacaaggag agagccggag tgctggttgg 600

gacaggaatg ggtggtctga ctgtcttctc tgacggggtt caatctctta tcgagaaggg 660

tcaccggaaa atcacccctt tcttcatccc ctatgccatt acaaacatgg ggtctgccct 720

gctcgctatt gaactcggtc tgatgggccc aaactattca atttccactg catgtgccac 780

ttccaactac tgcttccatg ctgctgctaa tcatatccgc cgtggtgagg ctgatcttat 840

gattgctgga ggcactgagg ccgcaatcat tccaattggg ttgggaggct ttgtggcttg 900

cagggctctg tctcaaagga acgatgaccc tcagactgcc tctaggccct gggataaaga 960

ccgtgatggt tttgtgatgg gtgaaggtgc tggagtgttg gtgctggaga gcttggaaca 1020

tgcaatgaaa cgaggagcac ctattattgc agagtatttg ggaggtgcaa tcaactgtga 1080

tgcttatcac atgactgacc caagggctga tggtctcggt gtctcctctt gcattgagag 1140

tagccttgaa gatgctggcg tctcacctga agaggtcaat tacataaatg ctcatgcgac 1200

ttctactcta gctggggatc tcgccgagat aaatgccatc aagaaggttt tcaagaacac 1260

aaaggatatc aaaattaatg caactaagtc aatgatcgga cactgtcttg gagcctctgg 1320

aggtcttgaa gctatagcga ctattaaggg aataaacacc ggctggcttc atcccagcat 1380

taatcaattc aatcctgagc catccgtgga gttcgacact gttgccaaca agaagcagca 1440

acacgaagtt aatgttgcga tctcgaattc atttggattc ggaggccaca actcagtcgt 1500

ggctttctcg gctttcaagc catgattacc catttcacaa ggcacttgtc attgagagta 1560

cggttgttcg tcaaacccat ttaggatact gttctatgta aaaaaaagta aggattatca 1620

ctttcccttc taatcctgtc tccagtttga gaatgaaatt atatttattt taaaaaaaaa 1680

aaaaaagggc ggccgctcta gaggatccaa gct 1713

<210> 10
<211> 465
<212> PRT
<213> Cuphea pulcherrima

<400> 10

Met His Ser Leu Gln Ser Pro Ser Leu Arg Ala Ser Pro Leu Asp Pro
1               5                   10                  15

Phe Arg Pro Lys Ser Ser Thr Val Arg Pro Leu His Arg Ala Ser Ile
            20                  25                  30

Pro Asn Val Arg Ala Ala Ser Pro Thr Val Ser Ala Pro Lys Arg Glu
        35                  40                  45

Thr Asp Pro Lys Lys Arg Val Val Ile Thr Gly Met Gly Leu Val Ser
    50                  55                  60

Val Phe Gly Ser Asp Val Asp Ala Tyr Tyr Asp Lys Leu Leu Ser Gly
65                  70                  75                  80

Glu Ser Gly Ile Gly Pro Ile Asp Arg Phe Asp Ala Ser Lys Phe Pro
                85                  90                  95

Thr Arg Phe Gly Gly Gln Ile Arg Gly Phe Asn Ser Met Gly Tyr Ile
            100                 105                 110

Asp Gly Lys Asn Asp Arg Arg Leu Asp Asp Cys Leu Arg Tyr Cys Ile
        115                 120                 125

Val Ala Gly Lys Lys Ser Leu Glu Asp Ala Asp Leu Gly Ala Asp Arg
    130                 135                 140

Leu Ser Lys Ile Asp Lys Glu Arg Ala Gly Val Leu Val Gly Thr Gly
145                 150                 155                 160

Met Gly Gly Leu Thr Val Phe Ser Asp Gly Val Gln Ser Leu Ile Glu
                165                 170                 175

Lys Gly His Arg Lys Ile Thr Pro Phe Phe Ile Pro Tyr Ala Ile Thr
            180                 185                 190

Asn Met Gly Ser Ala Leu Leu Ala Ile Glu Leu Gly Leu Met Gly Pro
        195                 200                 205

Asn Tyr Ser Ile Ser Thr Ala Cys Ala Thr Ser Asn Tyr Cys Phe His
    210                 215                 220

Ala Ala Ala Asn His Ile Arg Arg Gly Glu Ala Asp Leu Met Ile Ala
225                 230                 235                 240

Gly Gly Thr Glu Ala Ala Ile Ile Pro Ile Gly Leu Gly Gly Phe Val
                245                 250                 255

Ala Cys Arg Ala Leu Ser Gln Arg Asn Asp Asp Pro Gln Thr Ala Ser
            260                 265                 270

Arg Pro Trp Asp Lys Asp Arg Asp Gly Phe Val Met Gly Glu Gly Ala
        275                 280                 285

Gly Val Leu Val Leu Glu Ser Leu Glu His Ala Met Lys Arg Gly Ala
    290                 295                 300

Pro Ile Ile Ala Glu Tyr Leu Gly Gly Ala Ile Asn Cys Asp Ala Tyr
305                 310                 315                 320

His Met Thr Asp Pro Arg Ala Asp Gly Leu Gly Val Ser Ser Cys Ile
                325                 330                 335

Glu Ser Ser Leu Glu Asp Ala Gly Val Ser Pro Glu Glu Val Asn Tyr
            340                 345                 350

Ile Asn Ala His Ala Thr Ser Thr Leu Ala Gly Asp Leu Ala Glu Ile
        355                 360                 365

Asn Ala Ile Lys Lys Val Phe Lys Asn Thr Lys Asp Ile Lys Ile Asn

370                 375                 380

Ala Thr Lys Ser Met Ile Gly His Cys Leu Gly Ala Ser Gly Gly Leu
385                 390                 395                 400

Glu Ala Ile Ala Thr Ile Lys Gly Ile Asn Thr Gly Trp Leu His Pro
                405                 410                 415

Ser Ile Asn Gln Phe Asn Pro Glu Pro Ser Val Glu Phe Asp Thr Val
            420                 425                 430

Ala Asn Lys Lys Gln Gln His Glu Val Asn Val Ala Ile Ser Asn Ser
        435                 440                 445

Phe Gly Phe Gly Gly His Asn Ser Val Val Ala Phe Ser Ala Phe Lys
    450                 455                 460

Pro
465

<210>  11
<211>  1802
<212>  DNA
<213>  Cuphea pulcherrima

<400>  11
ggtcgaccca cgcgtccggg ctttccgacc acatttcatt tcttgcctcg ttatctccgc      60

cgctcctccg ccgtcgttcg ccgccgccgc catgcaatcc ctccactccc cttccctccg     120

cccctcccct ctcgagccct tccgcctcaa ttccccctcc tccgccgccg ctctccgccc     180

cctccgtcgc gccagcctcc ccgtcatccg tgctgccacc gcctccgccc ccaagcgcga     240

gtccgacccc aagaagcggg tcgtcatcac cggcatgggc ctcgtctccg tcttcggctc     300

cgacgtcgac gcctactacg acaagctgct ctccggcgag agcggcatca gcctaatcga     360

ccgcttcgac gcttccaaat tccccaccag gttcgccggc cagatccgtg gcttcaacgc     420

gacgggctac atcgacggca agaacgaccg gcggctcgac gattgcctcc gctactgcat     480

tgtcgccggc aagaaggctc tcgaagacgc cgatctcgcc ggccaatccc tctccaagat     540

tgataaggag agggccggag tgctagttgg aaccggtatg ggtggcctaa ctgtcttctc     600

tgacggggtt cagaatctca tcgagaaagg tcaccggaag atctccccgt tttcattccc     660

atatgccatt acaaacatgg ggtctgcgct gcttgccatc gatttgggtc tgatgggccc     720

aaactattcg atttcaactg catgtgctac ttccaactac tgcttttatg ctgccgccaa     780

tcatatccgc cgaggtgagg ctgacctgat gattgctgga ggaactgagg ctgcggtcat     840

tccaattggt ttaggaggat tcgttgcctg cagggcttta tctcaaagga atgatgatcc     900

tcagactgcc tcaaggccgt gggataagga ccgtgatggc tttgtgatgg gtgaaggggc 960

tggagtattg gttatggaga gcttggagca tgcaatgaaa cggggagcgc cgattattgc 1020

agaatatttg ggaggtgcag tcaactgtga tgcttatcat atgactgatc caagggctga 1080

tgggcttggt gtctcctcgt gcattgagag cagtctcgaa gatgccgggg tctcacctga 1140

agaggtcaat tacataaatg ctcatgcgac ttctactctt gctggggatc ttgccgagat 1200

aaatgccatt aagaaagttt tcaagaacac caaggaaatc aaaatcaatg caactaagtc 1260

aatgatcgga cactgtcttg gagcatcagg aggtcttgaa gccatcgcaa ccattaaggg 1320

aataaccacc ggctggcttc atcccagcat taatcaattt aatcccgagc catcggtgga 1380

cttcaacact gttgccaaca aaaagcagca acatgaagtg aacgtcgcta tctcgaattc 1440

ttttggattt ggagggcaca actcggttgt ggcattctca gctttcaagc catgaattct 1500

acttggttca aaatgcacac cagttgctga gatagggctt caacttgcag agcaattttt 1560

taaatgcctt gtcggaagag cgtaataccg gaataggtcg gtcctttgat agttcctcga 1620

agccatttag gatgatgttt tactgtaata atcgaagatg attcccattt taaatctagt 1680

ctctgattta tgtattagaa agaccaatga aagattttgt gtcatgtttg tgttgtcaat 1740

gttatttaag ataaagcaaa aaaaaaaaaa aagggcggcc gctctagagg atccagctta 1800

ct 1802

<210> 12
<211> 467
<212> PRT
<213> Cuphea pulcherrima

<400> 12

Met Gln Ser Leu His Ser Pro Ser Leu Arg Pro Ser Pro Leu Glu Pro
1               5                   10                  15

Phe Arg Leu Asn Ser Pro Ser Ser Ala Ala Ala Leu Arg Pro Leu Arg
            20                  25                  30

Arg Ala Ser Leu Pro Val Ile Arg Ala Ala Thr Ala Ser Ala Pro Lys
        35                  40                  45

Arg Glu Ser Asp Pro Lys Lys Arg Val Val Ile Thr Gly Met Gly Leu
    50                  55                  60

Val Ser Val Phe Gly Ser Asp Val Asp Ala Tyr Tyr Asp Lys Leu Leu
65                  70                  75                  80

Ser Gly Glu Ser Gly Ile Ser Leu Ile Asp Arg Phe Asp Ala Ser Lys
                85                  90                  95

Phe Pro Thr Arg Phe Ala Gly Gln Ile Arg Gly Phe Asn Ala Thr Gly
            100                 105                 110

Tyr Ile Asp Gly Lys Asn Asp Arg Arg Leu Asp Asp Cys Leu Arg Tyr
        115                 120                 125

Cys Ile Val Ala Gly Lys Lys Ala Leu Glu Asp Ala Asp Leu Ala Gly
    130                 135                 140

Gln Ser Leu Ser Lys Ile Asp Lys Glu Arg Ala Gly Val Leu Val Gly
145                 150                 155                 160

Thr Gly Met Gly Gly Leu Thr Val Phe Ser Asp Gly Val Gln Asn Leu
                165                 170                 175

Ile Glu Lys Gly His Arg Lys Ile Ser Pro Phe Phe Ile Pro Tyr Ala
            180                 185                 190

Ile Thr Asn Met Gly Ser Ala Leu Leu Ala Ile Asp Leu Gly Leu Met
        195                 200                 205

Gly Pro Asn Tyr Ser Ile Ser Thr Ala Cys Ala Thr Ser Asn Tyr Cys
    210                 215                 220

Phe Tyr Ala Ala Ala Asn His Ile Arg Arg Gly Glu Ala Asp Leu Met
225                 230                 235                 240

Ile Ala Gly Gly Thr Glu Ala Ala Val Ile Pro Ile Gly Leu Gly Gly
                245                 250                 255

Phe Val Ala Cys Arg Ala Leu Ser Gln Arg Asn Asp Asp Pro Gln Thr
            260                 265                 270

Ala Ser Arg Pro Trp Asp Lys Asp Arg Asp Gly Phe Val Met Gly Glu
        275                 280                 285

Gly Ala Gly Val Leu Val Met Glu Ser Leu Glu His Ala Met Lys Arg
    290                 295                 300

Gly Ala Pro Ile Ile Ala Glu Tyr Leu Gly Gly Ala Val Asn Cys Asp
305                 310                 315                 320

Ala Tyr His Met Thr Asp Pro Arg Ala Asp Gly Leu Gly Val Ser Ser
                325                 330                 335

Cys Ile Glu Ser Ser Leu Glu Asp Ala Gly Val Ser Pro Glu Glu Val
            340                 345                 350

Asn Tyr Ile Asn Ala His Ala Thr Ser Thr Leu Ala Gly Asp Leu Ala
        355                 360                 365

Glu Ile Asn Ala Ile Lys Lys Val Phe Lys Asn Thr Lys Glu Ile Lys
    370                 375                 380

Ile Asn Ala Thr Lys Ser Met Ile Gly His Cys Leu Gly Ala Ser Gly
385                 390                 395                 400

Gly Leu Glu Ala Ile Ala Thr Ile Lys Gly Ile Thr Thr Gly Trp Leu
                405                 410                 415

His Pro Ser Ile Asn Gln Phe Asn Pro Glu Pro Ser Val Asp Phe Asn
            420                 425                 430

Thr Val Ala Asn Lys Lys Gln Gln His Glu Val Asn Val Ala Ile Ser
        435                 440                 445

Asn Ser Phe Gly Phe Gly Gly His Asn Ser Val Val Ala Phe Ser Ala
    450                 455                 460

Phe Lys Pro
465

<210> 13
<211> 2369
<212> DNA
<213> Cuphea pulcherrima

<400> 13
   gtacgcctgc aggtaccggt ccggaattcc cgggtcgacc cacgcgtccg cataaaagag 60
   agagagaggg atccatcgaa tgcggccacc ctcctttcat cttcgattca ttaccatacc 120
   attccgctga tccattttcc gccttttccg ggtctttcat cccaaagggt atccttttct 180
   atcctatctt ctcaaagggt cagtcagttc cctccaatgc ctgccgcctc ttccctgctc 240
   gcttcccctc tctgtacgtg gctccttgcc gcctgcatgt ctacctcctt ccacccctcc 300
   gaccctcttc cgccttccat ctcctctcct cgccgacgcc tctcccgccg ccggattctc 360
   tcccaatgcg ccccactacc ttctgcttcc tccgccctcc gcggatccag tttccatacc 420
   ctcgtcacct cttacctcgc ctgcttcgag ccctgccatg actactatac atccgcatcc 480
   ttgttcggat ccagacccat tcgcaccacc cgcaggcacc ggaggctcaa tcgagcttcc 540
   ccttccaggg aggcaatggc cgtggctctg caacctgaac aggaagttac cacaaagaag 600
   aagccaagta tcaaacagcg gcgagtagtt gtgactggaa tgggtgtggt gactcctcta 660
   ggccatgacc ctgatgtttt ctacaataat ctgcttgatg gaacgagtgg cataagcgag 720
   atagagacct ttgattgtgc tcaatttcct acgagaattg ctggagagat caagtctttc 780
   tccacagatg gttgggtggc cccgaagctc tctaagagga tggacaagtt catgctatac 840
   atgctgaccg ctggcaagaa agcattaaca gatggtggaa tcaccgaaga tgtgatgaaa 900
   gagctagata aaagaaaatg cggagttctc attggctcag caatgggtgg aatgaaggta 960
   ttcaatgatg ccattgaagc cctaaggatt tcatataaga agatgaatcc cttttgtgta 1020
   cctttcgcta ccacaaatat gggatcagct atgcttgcaa tggacttggg atggatgggg 1080
   cccaactact cgatatctac tgcttgtgca acgagtaact tttgtataat gaatgctgcg 1140
   aaccatataa tcagaggcga agcagatgtg atgctttgcg ggggctcaga tgcggtaatc 1200
   atacctattg gtatgggagg ttttgttgca tgccgagctt tgtcccagag aaattccgac 1260
   cctactaaag cttcaagacc atgggacagt aatcgtgatg gatttgttat gggggaagga 1320
   gctggagtgc tactactaga ggagttggag catgcaaaga aaagaggtgc gactatttac 1380
   gcagaatttc taggtgggag tttcacttgc gatgcctacc acatgaccga gcctcaccct 1440
   gatggagctg gagtgattct ctgcatagag aaggctttgg ctcagtcagg agtctctagg 1500
   gaagacgtaa attacataaa tgcccatgcc acatccactc cggctggaga tatcaaagag 1560
   taccaagctc ttatccactg tttcggccaa aacagagagt taaaagttaa ttcaaccaaa 1620
   tcaatgattg gtcaccttct cggagcagcc ggtggtgtgg aagcagtttc agtagttcag 1680
   gcaataagga ctgggtggat ccatccgaat attaatttgg aaaacccaga tgaaggcgtg 1740
   gatacaaaat tgctcgtggg tcctaagaag gagagactga acgttaaggt cggtttgtct 1800
   aattcatttg ggtttggtgg gcacaactcg tccatactct tcgcccctta catctaggac 1860
   gtttccgtgt gtggaattct actcaacata tcaaagctga agttttgagg actccagcat 1920
   gttggtagct ccttacgtct ctagacatgc ccatgagttt tgtgtccgga gctttagtcg 1980
   gaaccatgac ggattgagta ctcatggcga cacttgatat actccttgct agaattgttg 2040
   gtagagcaat attcattatc tcatattttt tttttctctg aaatctccct ccttgcaata 2100
   gttgtacttt cgagcttttc atcgagtcag tgaagaagag aacaaagctg ttaactcggg 2160
   cacgtagtaa ccatttgccc tttgttttgc tctctatttc atcaccgttt tgtggtttta 2220
   aaatttgtaa aactagaaga ctggtttaga ttggtttgtt ttctcattga taattggggr 2280
   atgtatgttt tggaaataaa aaaaaaaaaa aaaaaaaaaa aaaaaaaaaa aaaaaaaaaa 2340
   aaaaaaaaaa agggcggccg ctctagagg 2369

<210> 14
<211> 546
<212> PRT
<213> Cuphea pulcherrima

<400> 14

Met Pro Ala Ala Ser Ser Leu Leu Ala Ser Pro Leu Cys Thr Trp Leu
1               5                   10                  15

Leu Ala Ala Cys Met Ser Thr Ser Phe His Pro Ser Asp Pro Leu Pro
            20                  25                  30

Pro Ser Ile Ser Ser Pro Arg Arg Arg Leu Ser Arg Arg Arg Ile Leu
        35                  40                  45

Ser Gln Cys Ala Pro Leu Pro Ser Ala Ser Ser Ala Leu Arg Gly Ser
    50                  55                  60

Ser Phe His Thr Leu Val Thr Ser Tyr Leu Ala Cys Phe Glu Pro Cys
65                  70                  75                  80

His Asp Tyr Tyr Thr Ser Ala Ser Leu Phe Gly Ser Arg Pro Ile Arg
                85                  90                  95

Thr Thr Arg Arg His Arg Arg Leu Asn Arg Ala Ser Pro Ser Arg Glu
            100                 105                 110

Ala Met Ala Val Ala Leu Gln Pro Glu Gln Glu Val Thr Thr Lys Lys
        115                 120                 125

Lys Pro Ser Ile Lys Gln Arg Arg Val Val Val Thr Gly Met Gly Val
    130                 135                 140

Val Thr Pro Leu Gly His Asp Pro Asp Val Phe Tyr Asn Asn Leu Leu
145                 150                 155                 160

Asp Gly Thr Ser Gly Ile Ser Glu Ile Glu Thr Phe Asp Cys Ala Gln
                165                 170                 175

Phe Pro Thr Arg Ile Ala Gly Glu Ile Lys Ser Phe Ser Thr Asp Gly
            180                 185                 190

Trp Val Ala Pro Lys Leu Ser Lys Arg Met Asp Lys Phe Met Leu Tyr
        195                 200                 205

Met Leu Thr Ala Gly Lys Lys Ala Leu Thr Asp Gly Gly Ile Thr Glu
    210                 215                 220

Asp Val Met Lys Glu Leu Asp Lys Arg Lys Cys Gly Val Leu Ile Gly
225                 230                 235                 240

Ser Ala Met Gly Gly Met Lys Val Phe Asn Asp Ala Ile Glu Ala Leu
                245                 250                 255

Arg Ile Ser Tyr Lys Lys Met Asn Pro Phe Cys Val Pro Phe Ala Thr
            260                 265                 270

Thr Asn Met Gly Ser Ala Met Leu Ala Met Asp Leu Gly Trp Met Gly
        275                 280                 285

Pro Asn Tyr Ser Ile Ser Thr Ala Cys Ala Thr Ser Asn Phe Cys Ile
    290                 295                 300

Met Asn Ala Ala Asn His Ile Ile Arg Gly Glu Ala Asp Val Met Leu
305                 310                 315                 320

Cys Gly Gly Ser Asp Ala Val Ile Ile Pro Ile Gly Met Gly Gly Phe
                325                 330                 335

Val Ala Cys Arg Ala Leu Ser Gln Arg Asn Ser Asp Pro Thr Lys Ala
            340                 345                 350

Ser Arg Pro Trp Asp Ser Asn Arg Asp Gly Phe Val Met Gly Glu Gly
        355                 360                 365

Ala Gly Val Leu Leu Leu Glu Glu Leu Glu His Ala Lys Lys Arg Gly
    370                 375                 380

Ala Thr Ile Tyr Ala Glu Phe Leu Gly Gly Ser Phe Thr Cys Asp Ala
385                 390                 395                 400

Tyr His Met Thr Glu Pro His Pro Asp Gly Ala Gly Val Ile Leu Cys
                405                 410                 415

Ile Glu Lys Ala Leu Ala Gln Ser Gly Val Ser Arg Glu Asp Val Asn
            420                 425                 430

Tyr Ile Asn Ala His Ala Thr Ser Thr Pro Ala Gly Asp Ile Lys Glu
        435                 440                 445

Tyr Gln Ala Leu Ile His Cys Phe Gly Gln Asn Arg Glu Leu Lys Val
    450                 455                 460

Asn Ser Thr Lys Ser Met Ile Gly His Leu Leu Gly Ala Ala Gly Gly
465                 470                 475                 480

Val Glu Ala Val Ser Val Val Gln Ala Ile Arg Thr Gly Trp Ile His
                485                 490                 495

Pro Asn Ile Asn Leu Glu Asn Pro Asp Glu Gly Val Asp Thr Lys Leu
            500                 505                 510

Leu Val Gly Pro Lys Lys Glu Arg Leu Asn Val Lys Val Gly Leu Ser
        515                 520                 525

Asn Ser Phe Gly Phe Gly Gly His Asn Ser Ser Ile Leu Phe Ala Pro
    530                 535                 540

Tyr Ile
545

<210>  15
<211>  2372
<212>  DNA
<213>  Cuphea pulcherrima

<220>
<221>  misc_feature
<222>  (1)..(2300)
<223>  unsure at all n locations

<400>  15
acntggtccg gaattcccgg gtcgacccac gcgtccgcga cgccaaccca caccaaactt      60

cctcagcttc tcttctcaag acggacgcca ttggcagcag acagacagac agacagaccc     120

ataaaagaga gagagaggga tccatcgaat gcggccaccc tcctttcatc ttcgattcat     180

taccatacca ttccgctgat ccattttccg ccttttccgg gtctttcatc ccaaagggta     240

tccttttcta tcctatcttc tcaaagggtc agtcagttcc ctccaatgcc tgccgcctct     300

tccctgctcg cttcccctct ctgtacgtgg ctccttgccg cctgcatgtc tacctccttc     360

cacccctccg accctcttcc gccttccatc tcctctcctc gccgacgcct ctcccgccgc     420

cggattctct cccaatgcgc cccactacct tctgcttcct ccgccctccg cggatccagt     480

ttccataccc tcgtcacctc ttacctcgcc tgcttcgagc cctgccatga ctactataca     540

tccgcatcct tgttcggatc cagacccatt cgcaccaccc gcaggcaccg gaggctcaat     600

cgagcttccc cttccagggg aggcaatggc cgtggctctg caacctgaac aggaagttac     660

cacaaagaag aagccaagta tcaaacagcg gcgagtagtt gtgactggaa tgggtgtggt     720

gactcctcta ggccatgaac ctgatgtttt tctacaataa tctgcttgat ggaacgagtg     780

gcataagcga gatagagacc tttgattgtg ctcaatttcc tacgagaatt gctggagaga     840

tcaagtcttt ctccacagat ggttgggtgg ccccgaagct ctctaagagg atggacaagt     900

tcatgctata catgctgact gctggcaaga aagcattaac agatggtgga atcaccgaag     960

atgtgatgaa agagctagat aaaagaaaat gcggagttct cattggctca gcaatgggtg    1020

gaatgaaggt attcaatgat gccattgaag ccctaaggat ttcatataag aagatgaatc 1080

ccttttgtgt acctttcgct accacaaata tgggatcagc tatgcttgca atggacttgg 1140

gatggatggg gcccaactac tcgatatcta ctgcttgtgc aacgagtaac ttttgtataa 1200

tgaatgctgc gaaccatata atcagaggcg aagcagatgt gatgctttgc gggggctcag 1260

atgcggtaat catacctatt ggtatgggag gttttgttgc atgccgagct ttgtcccaga 1320

gaaattccga ccctactaaa gcttcaagac catgggacag taatcgtgat ggatttgtta 1380

tgggggaagg agctggagtg ctactactag aggagttgga gcatgcaaag aaaagaggtg 1440

cgactattta cgcagaattt ctaggtggga gtttcacttg cgatgcctac cacatgaccg 1500

agcctcaccc tgatggagct ggagtgattc tctgcataga gaaggctttg gctcagtcag 1560

gagtctctag ggaagacgta aattacataa atgcccatgc cacatccact ccggctggag 1620

atatcaaaga gtaccaagct cttatccact gtttcggcca aaacagagag ttaaaagtta 1680

attcaaccaa atcaatgatt ggtcaccttc tcggagcagc cggtggtgtg gaagcagttt 1740

cagtagttca ggcaataagg actgggtgga tccatccgaa tattaatttg gaaaacccag 1800

atgaaggcgt ggatacaaaa ttgctcgtgg gtcctaagaa ggagagactg aacgttaagg 1860

tcggtttgtc taattcattt gggtttggtg ggcacaactc gtccatactc ttcgcccctt 1920

acatctagga cgtttcgtgt gtggaattct actcaacata tcaaagctga agttttgagg 1980

actccagcat gttggtagct ccttacgtct ctagacatgc ccatgagttt tgtgtccgga 2040

gctttagtcg gaaccatgac ggattgagta ctcatggcga cacttgatat actccttgct 2100

agaattgttg gtagagcaat attcattatc tcatattttt tttttctctg aaatctccct 2160

ccttgcaata gttgtacttt cgagcttttc atcgagtcag tgaagaagag aacaaagctg 2220

ttaactcggg cacgtagtaa ccatttgccc tttgttttgc tctctatttc atcaccgttt 2280

tgtggtttta aaatttgtaa aactagaaga ctggtttaga ttggtttgtt ttctcaaaaa 2340

aaaaaaaaaa gggcggccgc tctagaggat cc 2372

<210> 16
<211> 1580
<212> DNA
<213> Cuphea hookeriana

<220>
<221> misc_feature
<222> (1)..(1578)
<223> unsure at all n locations

<400> 16
cctgaatcgg attcaagaga gagtttcgtt gctgggatgg cgaatgcatc tgggtttctg 60

ggttcttcag ttcctgccct gagaagggca actcagcatt cgatttcatc gtctcgtgga 120
tcttcctcgg agtttgtctc caaaagggtg ttttgctgta gtgccgttca ggattctgac 180
aggcagtctt tgggtgattc tcgctcgccg aggcttgtga gtagaggatg caaattaatt 240
ggatctggtt ctgctatacc agctcttcaa gtctcaaatg atgatcttgc taaaattgtc 300
gacaccaatg atgaatggat tactgtccga acggggatcc gcaaccgaag ggttctctca 360
ggtaaagata gtcttacaaa tttagcatca gaggcagcaa ggaaagctct agagatggca 420
caggtagacg caaatgatgt ggatatggtt ttgatgtgta cttctacccc tgaggacctt 480
ttcggcagtg ctcctcagat atcgaaagca cttggctgca aaaagaatcc tttgtcttac 540
gacattaccg ctgcatgcag tggatttgtg ttgggtttag tctcagctgc ttgccacatt 600
agaggtgggg gttttaacaa tattctagtg attggtgctg attctctttc tcggtatgtt 660
gactggaccg atcggggaac atgtattctc tttggagatg ctgctggagc tgtagtggtg 720
cagtcatgtg atgctgagga agatgggctc tttgcttttg atttgcatag cgatggagat 780
gggcaaaggc atctaaaagc tgcaatcaaa gaagatgaag ttgataaagc cctgggacat 840
aatgggtcca tcagagattt tccaccaagg cgttcttcat actcttgcat ccaaatgaac 900
ggtaaagagg tattccgctt tgcttgccgc tctgtgcctc agtcaatcga atcagcactt 960
ggaaaggccg gtcttaatgg atccaacatc gactggttgc tgcttcatca ggcaaatcag 1020
aggatcattg atgcagtagc aacacgtcta gaggttcctc aagaacgaat tatctcaaac 1080
ttggcaaatt acgggaacac tagtgcggca tccattccct tggcactaga cgaagctgtg 1140
aggagtggaa atgtgaagcc gggtcacgtg attgcaaccg caggatttgg cgccggactc 1200
acatggggtt ctgctattat caggtgggga taagactgaa gccgagccag cactgcagct 1260
tcctctcaaa ccgatgtttc acgaaatttt gcttccatga ccanaaaaag aagaagtcag 1320
tcttttatgg agcaagcaac acgacacgat cttcatcaca ttgccctttt tcgttcccct 1380
tttccattag tttgatgatt ttgctgacaa tacaataccc atagtttctt ttgtccccaa 1440
taagttattt gtttcttgtt taattgttca gcttttactt cattttgtct cgggacattg 1500
gagatgacag cataaacatc atgtttatat tttgctaaaa aaaaaaaaaa aaaaaaaaaa 1560
aaaaaaaaaa aaaaaaaaaa 1580

<210> 17
<211> 398
<212> PRT
<213> Cuphea hookeriana

<400> 17

Met Ala Asn Ala Ser Gly Phe Leu Gly Ser Ser Val Pro Ala Leu Arg
1               5                   10                  15

Arg Ala Thr Gln His Ser Ile Ser Ser Ser Arg Gly Ser Ser Ser Glu
            20                  25                  30

Phe Val Ser Lys Arg Val Phe Cys Cys Ser Ala Val Gln Asp Ser Asp
        35                  40                  45

Arg Gln Ser Leu Gly Asp Ser Arg Ser Pro Arg Leu Val Ser Arg Gly
    50                  55                  60

Cys Lys Leu Ile Gly Ser Gly Ser Ala Ile Pro Ala Leu Gln Val Ser
65                  70                  75                  80

Asn Asp Asp Leu Ala Lys Ile Val Asp Thr Asn Asp Glu Trp Ile Thr
                85                  90                  95

Val Arg Thr Gly Ile Arg Asn Arg Arg Val Leu Ser Gly Lys Asp Ser
            100                 105                 110

Leu Thr Asn Leu Ala Ser Glu Ala Ala Arg Lys Ala Leu Glu Met Ala
        115                 120                 125

Gln Val Asp Ala Asn Asp Val Asp Met Val Leu Met Cys Thr Ser Thr
    130                 135                 140

Pro Glu Asp Leu Phe Gly Ser Ala Pro Gln Ile Ser Lys Ala Leu Gly
145                 150                 155                 160

Cys Lys Lys Asn Pro Leu Ser Tyr Asp Ile Thr Ala Ala Cys Ser Gly
                165                 170                 175

Phe Val Leu Gly Leu Val Ser Ala Ala Cys His Ile Arg Gly Gly Gly
            180                 185                 190

Phe Asn Asn Ile Leu Val Ile Gly Ala Asp Ser Leu Ser Arg Tyr Val
        195                 200                 205

Asp Trp Thr Asp Arg Gly Thr Cys Ile Leu Phe Gly Asp Ala Ala Gly
    210                 215                 220

Ala Val Val Val Gln Ser Cys Asp Ala Glu Glu Asp Gly Leu Phe Ala
225                 230                 235                 240

Phe Asp Leu His Ser Asp Gly Asp Gly Gln Arg His Leu Lys Ala Ala
                245                 250                 255

Ile Lys Glu Asp Glu Val Asp Lys Ala Leu Gly His Asn Gly Ser Ile
            260                 265                 270

Arg Asp Phe Pro Pro Arg Arg Ser Ser Tyr Ser Cys Ile Gln Met Asn
        275                 280                 285

Gly Lys Glu Val Phe Arg Phe Ala Cys Arg Ser Val Pro Gln Ser Ile
    290                 295                 300

Glu Ser Ala Leu Gly Lys Ala Gly Leu Asn Gly Ser Asn Ile Asp Trp
305                 310                 315                 320

Leu Leu Leu His Gln Ala Asn Gln Arg Ile Ile Asp Ala Val Ala Thr
                325                 330                 335

Arg Leu Glu Val Pro Gln Glu Arg Ile Ile Ser Asn Leu Ala Asn Tyr
            340                 345                 350

Gly Asn Thr Ser Ala Ala Ser Ile Pro Leu Ala Leu Asp Glu Ala Val
        355                 360                 365

Arg Ser Gly Asn Val Lys Pro Gly His Val Ile Ala Thr Ala Gly Phe
    370                 375                 380

Gly Ala Gly Leu Thr Trp Gly Ser Ala Ile Ile Arg Trp Gly
385                 390                 395

??

??

??

??

1

1

## Description

### Field of Invention

The present invention is directed to genes encoding plant fatty acid synthase enzymes relevant to fatty acid synthesis in plants, and to methods of using such genes in combination with genes encoding plant medium-chain preferring thioesterase proteins. Such uses provide a method to increase the levels of medium-chain fatty acids that may be produced in seed oils of transgenic plants.

### Background

Higher plants synthesize fatty acids via a common metabolic pathway. In developing seeds, where fatty acids attached to triglycerides are stored as a source of energy for further germination, the fatty acid synthesis pathway is located in the plastids. The first step is the formation of acetyl-ACP (acyl carrier protein) from acetyl-CoA and ACP catalyzed by a short chain preferring condensing enzyme, ß-ketoacyl-ACP synthase (KAS) III. Elongation of acetyl-ACP to 16- and 18- carbon fatty acids involves the cyclical action of the following sequence of reactions: condensation with a two-carbon unit from malonyl-ACP to form a longer ß-ketoacyl-ACP (ß-ketoacyl-ACP synthase), reduction of the keto-function to an alcohol (ß-ketoacyl-ACP reductase), dehydration to form an enoyl-ACP (ß-hydroxyacyl-ACP dehydrase), and finally reduction of the enoyl-ACP to form the elongated saturated acyl-ACP (enoyl-ACP reductase). ß-ketoacyl-ACP synthase I (KAS I), is primarily responsible for elongation up to palmitoyl-ACP (C16:0), whereas ß-ketoacyl-ACP synthase II (KAS II) is predominantly responsible for the final elongation to stearoyl-ACP (C18:0).

Genes encoding peptide components of ß-ketoacyl-ACP synthases I and II have been cloned from a number of higher plant species, including castor (*Ricinus communis*) and *Brassica* species (USPN 5,510,255). KAS I activity was associated with a single synthase protein factor having an approximate molecular weight of 50 kD (synthase factor B) and KAS II activity was associated with a combination of two synthase protein factors, the 50 kD synthase factor B and a 46 kd protein designated synthase factor A. Cloning and sequence of a plant gene encoding a KAS III protein has been reported by Tai and Jaworski (*Plant Physiol.* (1993) *103*:1361-1367).

The end products of plant fatty acid synthetase activities are usually 16- and 18-carbon fatty acids. There are, however, several plant families that store large amounts of 8- to 14-carbon (medium-chain) fatty acids in their oilseeds. Recent studies with *Umbellularia* californica (California bay), a plant that produces seed oil rich in lauric acid (12;0), have demonstrated the existence of a medium-chain-specific isozyme of acyl-ACP thioesterase in the seed plastids. Subsequent purification of the 12:0-ACP thioesterase from *Umbellularia californica* led to the cloning of a thioesterase cDNA which was expressed in seeds of *Arabidopsis* and *Brassica* resulting in a substantial accumulation of lauric acid in the triglyceride pools of these transgenic seeds (USPN 5,512,482). These results and subsequent studies with medium-chain thioesterases from other plant species have confirmed the chain-length-determining role of acyl-ACP thioesterases during de novo fatty acid biosynthesis (T. Voelker (1996) Genetic *Engineering*, Ed. J. K. Setlow, Vol. 18, pgs. 111-133).

### DESCRIPTION OF THE FIGURES

Figure 1. DNA and translated amino acid sequence of *Cuphea hookeriana* KAS factor B clone chKAS B-2 are provided.
Figure 2. DNA and translated amino acid sequence of *Cuphea hookeriana* KAS factor B clone chKAS B-31-7 are provided.
Figure 3. DNA and translated amino acid sequence of *Cuphea hookeriana* KAS factor A clone chKAS A-2-7 are provided.
Figure 4. DNA and translated amino acid sequence of *Cuphea hookeriana* KAS factor A clone chKAS A-1-6 are provided.
Figure 5. DNA and translated amino acid sequence of *Cuphea pullcherrima* KAS factor B clone cpuKAS B/7-8 are provided.
Figure 6. DNA and translated amino acid sequence of *Cuphea pullcherrima* KAS factor B clone cpuKAS B/8-7A are provided.
Figure 7. DNA and translated amino acid sequence of *Cuphea pullcherrima* KAS factor A clone cpuKAS A/p7-6A are provided..
Figure 8. Preliminary DNA sequence of *Cuphea pullcherrima* KAS factor A clone cpuKAS A/p8-9A is provided.
Figure 9. DNA and translated amino acid sequence of *Cuphea hookeriana* KASIII clone chKASIII-27 are provided.
Figure 10. The activity profile for purified cpuKAS B/8-7A using various acyl-ACP substrates is provided.
Figure 11. The activity profile for purified chKAS A-2-7 and chKAS A-1-6 using various acyl-ACP substrates is provided.
Figure 12. The activity profile for purified castor KAS factor A using various acyl-ACP substrates is provided.
Figure 13. The activity profile for purified castor KAS factor B using various acyl-ACP substrates is provided.
Figure 14. A graph showing the number of plants arranged according to C8:0 content for transgenic plants containing CpFatB1 versus transgenic plants containing CpFatB1 + chKAS A-2-7 is provided.
Figure 15. Graphs showing the %C10/%C8 ratios in transgenic plants containing ChFatB2 (4804-22-357) and in plants resulting from crosses between 4804-22-357 and 5401-9 (chKAS A-2-7 plants) are provided.
Figure 16. Graphs showing the %C10 + %C8 contents in transgenic plants containing ChFatB2 (4804-22-357) and in plants resulting from crosses between 4804-22-357 and 5401-9 (chKAS A-2-7 plants) are provided.
Figure 17. Graphs showing the %C10/%C8 ratios in transgenic plants containing ChFatB2 (4804-22-357) and in plants resulting from crosses between 4804-22-357 and 5413-17 (chKAS A-2-7 + CpFatB1 plants) are provided.
Figure 18. Graphs showing the %C10 + %C8 contents in transgenic plants containing ChFatB2 (4804-22-357) and in plants resulting from crosses between 4804-22-357 and 5413-17 (chKAS A-2-7 + CpFatB1 plants) are provided.
Figure 19. Graphs showing the %C12:0 in transgenic plants containing Uc FatB1 (LA86DH186) and in plants resulting from crosses with wild type (X WT) and with lines expressing Ch KAS A-2-7.
Figure 20. Graph showing the relative proportions of C12:0 and C14:0 fatty acids in the seeds of transgenic plants containing Uc FatB1 (LA8617H186) and in plants resulting from crosses with wild type (X WT) and with lines expressing Ch KAS A-2-7.
Figure 21. Graphs showing the %C18:0 in transgenic plants containing Garm FatB1 (5266) and in seeds of plants resulting from crosses with wild type (X WT) and with lines expressing Ch KAS A-2-7.
Figure 22. The activity profile of Ch KAS A in protein extracts from transgenic plants containing Ch KAS A-2-7. Extracts were preptreated with the indicated concentrations of cerulenin.

### SUMMARY OF THE INVENTION

By this invention, compositions and methods of use related to ß-ketoacyl-ACP synthase (KAS) are provided. Also of interest are methods and compositions of amino acid and nucleic acid sequences related to biologically active plant synthase(s).

In particular, genes encoding KAS protein factors A and B from *Cuphea* species are provided. The KAS genes are of interest for use in a variety of applications, and may be used to provide synthase I and/or synthase II activities in transformed host cells, including bacterial cells, such as *E. coli*, and plant cells. Synthase activities are distinguished by the preferential activity towards longer and shorter acyl-ACPs as well as by the sensitivity towards the KAS specific inhibitor, cerulenin Synthase protein preparations having preferential activity towards medium chain length acyl-ACPs are synthase I-type or KAS I. The KAS I class is sensitive to inhibition by cerulenin at concentrations as low as 1µM. Synthases having preferential activity towards longer chain length acyl-ACPs are synthase II-type or KAS II. The KAS enzymes of the II-type are also sensitive to cerulenin, but at higher concentrations (50µM). Synthase III-type enzymes have preferential activity towards short chain length acyl-ACPs and are insensitive to cerulenin inhibition.

Nucleic acid sequences encoding a synthase protein may be employed in nucleic acid constructs to modulate the amount of synthase activity present in the host cell, especially the relative amounts of synthase I-type, synthase II-type and synthase III-type activity when the host cell is a plant host cell. In addition, nucleic acid constructs may be designed to decrease expression of endogenous synthase in a plant cell as well. One example is the use of an anti-sense synthase sequence under the control of a promoter capable of expression in at least those plant cells which normally produce the enzyme.

Of particular interest in the present invention is the coordinate expression of a synthase protein with the expression of thioesterase proteins. For example, coordinated expression of synthase factor A and a medium-chain thioesterase provides a method for increasing the level of medium-chain fatty acids which may be harvested from transgenic plant seeds. Furthermore, coordinated expression of a synthase factor A gene with plant medium-chain thioesterase proteins also provides a method by which the ratios of various medium-chain fatty acids produced in a transgenic plant may be modified. For example, by expression of a synthase factor A, it is possible to increase the ratio of C10/C8 fatty acids which are produced in plant seed oils as the result of expression of a thioesterase having activity on C8 and C10 fatty acids.

### DETAILED DESCRIPTION OF THE INVENTION

A plant synthase factor protein of this invention includes a sequence of amino acids or polypeptide which is required for catalyzation of a condensation reaction between an acyl-ACP having a chain length of C₂ to C₁₆ and malonyl-ACP in a plant host cell. A particular plant synthase factor protein may be capable of catalyzing a synthase reaction in a plant host cell (for example as a monomer or homodimer) or may be one component of a multiple peptide enzyme which is capable of catalyzing a synthase reaction in a plant host cell, i.e. one peptide of a heterodimer.

Synthase I (KAS I) demonstrates preferential activity towards acyl-ACPs having shorter carbon chains, C₂-C₁₄ and is sensitive to inhibition by cerulenin at concentrations of 1µM. Synthase II (KAS II) demonstrates preferential activity towards acyl-ACPs having longer carbon chains, C₁₄-C₁₆, and is inhibited by concentrations of cerulenin (50µM)· Synthase III demonstrates preferential activity towards acyl-CoAs having very short carbon chains, C₂ to C₆, and is insensitive to inhibition by cerulenin.

Synthase factors A and B, and synthase III proteins obtained from medium-chain fatty acid producing plant species of the genus *Cuphea* are described herein.. As described in the following Examples, synthase A from C. *hookeriana* is naturally expressed at a high level and only in the seeds. C. *hookeriana* synthase B is expressed at low levels in all tissues examined. Expression of synthase A and synthase B factors in *E*. *coli* and purification of the resulting proteins is employed to determine activity of the various synthase factors. Results of these analyses indicate that synthase factor A from *Cuphea hookeriana* has the greatest activity on 6:0-ACP substrates, whereas synthase factor B from *Cuphea pullcherrima* has greatest activity on 14:0-ACP. Similar studies with synthase factors A and B from castor demonstrate similar activity profiles between the factor B synthase proteins from *Cuphea* and castor. The synthase A clone from castor, however, demonstrates a preference for 14:0-ACP substrate.

Expression of a *Cuphea hookeriana* KAS A protein in transgenic plant seeds which normally do not produce medium-chain fatty acids does not result in any detectable modification of the fatty acid types and contents produced in such seeds. However, when *Cuphea hookeriana* KAS A protein is expressed in conjunction with expression of a medium-chain acyl-ACP thioesterase capable of providing for production of C8 and C10 fatty acids in plant seed oils, increases in the levels of medium-chain fatty acids over the levels obtainable by expression of the medium-chain thioesterase alone are observed. In addition, where significant amounts of C8 and C10 fatty acids are produced as the result of medium-chain thioesterase expression, co-expression of a *Cuphea* KAS A protein also results in an alteration of the proportion of the C8 and C10 fatty acids that are obtained. For example, an increased proportion of C10 fatty acids may be obtained by co-expression of *Cuphea hookeriana* ChFatB2 thioesterase and a chKAS A synthase factor proteins.

Furthermore, when *Cuphea hookeriana* KAS A protein is expressed in conjunction with expression of a medium-chain acyl-ACP thioesterase capable of providing for production of C12 fatty acids in plant seed oils, increases in the levels of medium-chain fatty acids over the levels obtainable by expression of the medium-chain thioesterase alone are also observed. In addition, where significant amounts of C12 and C14 fatty acids are produced as the result of medium-chain thioesterase expression, co-expression of a *Cuphea* KAS A protein also results in an alteration of the proportion of the C12 and C14 fatty acids that are obtained. For example, an increased proportion of C12 fatty acids may be obtained by co-expression of *Uc* FatB1 thioesterase and a chKAS A synthase factor proteins.

However, when *Cuphea hookeriana* KAS A protein is expressed in conjunction with the expression of a long-chain acyl-ACP thioesterase capable of providing for production of C18 and C18:1 fatty acids in plant seed oils, no effect on the production of long chain fatty acids was observed. Furthermore, when plants transformed to express a long chain acyl-ACP thioesterase from mangosteen (*Garm*FatA1, U.S. Patent Application No. 08/440,845), which preferentially hydrolyzes C18:0 and C18:1 fatty acyl-ACPs, are crossed with nontransformed control plants, a significant reduction in the levels of C18:0 is obtained. Similar reductions are also observed in the levels of C18:0 in the seeds of plants resulting from crosses between plants transformed to express the *Garm*FatA1 and plants expressing the *Cuphea hookeriana* KAS A protein.

Thus, the instant invention provides methods of increasing and/or altering the medium-chain fatty acid compositions in transgenic plant seed oils by co-expression of medium-chain acyl-ACP thioesterases with synthase factor proteins. Furthermore, various combinations of synthase factors and medium-chain thioesterases may be achieved depending upon the particular fatty acids desired. For example, for increased production of C14 fatty acids, synthase protein factors may be expressed in combination with a C14 thioesterase, for example from Cuphea *palustris* or nutmeg may be employed (WO 96/23892). In addition, thioesterase expression may be combined with a number of different synthase factor proteins for additional effects on medium-chain fatty acid composition.

Synthases of use in the present invention include modified amino acid sequences, such as sequences which have been mutated, truncated, increased and the like, as well as such sequences which are partially or wholly artificially synthesized. The synthase protein encoding sequences provided herein may be employed in probes for further screening or used in genetic engineering constructs for transcription or transcription and translation in host cells, especially plant host cells. One skilled in the art will readily recognize that antibody preparations, nucleic acid probes (DNA and RNA) and the like may be prepared and used to screen and recover synthases and/or synthase nucleic acid sequences from other sources. Typically, a homologously related nucleic acid sequence will show at least about 60% homology, and more preferably at least about 70% homology, between the *R.. communis* synthase and the given plant synthase of interest, excluding any deletions which may be present Homology is determined upon comparison of sequence information, nucleic acid or amino acid, or through hybridization reactions.

Recombinant constructs containing a nucleic acid sequence encoding a synthase protein factor or nucleic acid sequences encoding a synthase protein factor and a medium-chain acyl-ACP thioesterase may be prepared by methods well known in the art. Constructs may be designed to produce synthase in either prokaryotic or eukaryotic cells. The increased expression of a synthase in a plant cell, particularly in conjunction with expression of medium-chain thioesterases, or decreasing the amount of endogenous synthase observed in plant cells are of special interest.

Synthase protein factors may be used, alone or in combination, to catalyze the elongating condensation reactions of fatty acid synthesis depending upon the desired result. For example, rate influencing synthase activity may reside in synthase I-type, synthase II-type, synthase III-type or in a combination of these enzymes. Furthermore, synthase activities may rely on a combination of the various synthase factors described herein.

Constructs which contain elements to provide the transcription and translation of a nucleic acid sequence of interest in a host cell are "expression cassettes". Depending upon the host, the regulatory regions will vary, including regions from structural genes from viruses, plasmid or chromosomal genes, or the like. For expression in prokaryotic or eukaryotic microorganisms, particularly unicellular hosts, a wide variety of constitutive or regulatable promoters may be employed. Among transcriptional initiation regions which have been described are regions from bacterial and yeast hosts, such as E. *coli, B. subtilis*, *Saccharomyces cerevisiae,* including genes such as ß-galactosidase, T7 polymerase, trp-lac (tac), trp E and the like.

An expressi.on cassette for expression of synthase in a plant cell will include, in the 5' to 3' direction of transcription, a transcription and translation initiation control regulatory region (also known as a "promoter") functional in a plant cell, a nucleic acid sequence encoding a synthase, and a transcription termination region. Numerous transcription initiation regions are available which provide for a wide variety of constitutive or regulatable, e.g., inducible, transcription of the desaturase structural gene. Among transcriptional initiation regions used for plants are such regions associated with cauliflower mosaic viruses (35S, 19S), and structural genes such as for nopaline synthase or mannopine synthase or napin and ACP promoters, etc. The transcription/ translation initiation regions corresponding to such structural genes are found immediately 5' upstream to the respective start codons. Thus, depending upon the intended use, different promoters may be desired.

of special interest in this invention are the use of promoters which are capable of preferentially expressing the synthase in seed tissue, in particular, at early stages of seed oil formation. Examples of such seed-specific promoters include the region immediately 5' upstream of a napin or seed ACP genes such as described in USPN 5,420,034, desaturase genes such as described in Thompson *et al* (*Proc. Nat. Acad. Sci.* (1991) *88:2578-2582),* or a Bce-4 gene such as described in USPN 5,530,194. Alternatively, the use of the 5' regulatory region associated with the plant synthase structural gene, i.e., the region immediately 5' upstream to a plant synthase structural gene and/or the transcription termination regions found immediately 3' downstream to the plant synthase structural gene, may often be desired. In general, promoters will be selected based upon their expression profile which may change given the particular application,

In addition, one may choose to provide for the transcription or transcription and translation of one or more other sequences of interest in concert with the expression or anti-sense of the synthase sequence, particularly medium-chain plant thioesterases such as described in USPN 5,512,482, to affect alterations in the amounts and/or composition of plant oils.

When one wishes to provide a plant transformed for the combined effect of more than one nucleic acid sequence of interest, a separate nucleic acid construct may be provided for each or the constructs may both be present on the same plant transformation construct. The constructs may be introduced into the host cells by the same or different methods, including the introduction of such a trait by crossing transgenic plants via traditional plant breeding methods, so long as the resulting product is a plant having both characteristics integrated into its genome.

Normally, included with the DNA construct will be a structural gene having the necessary regulatory regions for expression in a host and providing for selection of transformed cells. The gene may provide for resistance to a cytotoxic agent, *e.g.* antibiotic, heavy metal, toxin, etc., complementation providing prototrophy to an auxotrophic host, viral immunity or the like. Depending upon the number of different host species into which the expression construct or components thereof are introduced, one or more markers may be employed, where different conditions for selection are used for the different hosts.

The manner in which the DNA construct is introduced into the plant host is not critical to this invention. Any method which provides for efficient transformation may be employed. Various methods for plant cell transformation include the use of Ti- or Ri-plasmids, microinjection, electroporation, liposome fusion, DNA bombardment or the like. In many instances, it will be desirable to have the construct bordered on one or both sides by T-DNA, particularly having the left and right borders, more particularly the right border. This is particularly useful when the construct uses *A. tumefaciens* or *A*. *rhizogenes* as a mode for transformation, although the T-DNA borders may find use with other modes of transformation.

The expression constructs may be employed with a wide variety of plant life, particularly plant life involved in the production of vegetable oils. These plants include, but are not limited to rapeseed, peanut, sunflower, safflower, cotton, soybean, corn and oilseed palm.

For transformation of plant cells using *Agrobacterium,* explants may be combined and incubated with the transformed *Agrobacterium* for sufficient time for transformation, the bacteria killed, and the plant cells cultured in an appropriate selective medium. Once callus forms, shoot formation can be encouraged by employing the appropriate plant hormones in accordance with known methods and the shoots transferred to rooting medium for regeneration of plants. The plants may then be grown to seed and the seed used to establish repetitive generations and for isolation of vegetable oils.

The invention now being generally described, it will be more readily understood by reference to the following examples which are included for purposes of illustration only and are not intended to limit the present invention.

### EXAMPLES

### Example 1 Cuphea KAS Factor A and B Gene Cloning

Total RNA isolated from developing seeds of Cuphea *hookeriana and Cuphea pullcherrima* was used for cDNA synthesis in commercial 1-based cloning vectors. For cloning each type of KAS gene, approximately 400,000-500,000 unamplified recombinant phage were plated and the plaques transferred to nitrocellulose. For KAS factor B cloning from C. *hookeriana,* a mixed probe containing *Brassica napus* KAS factor B and *Ricinus communis* (Castor) KAS factor B radiolabeled cDNA's was used. Similarly, a mixed probe containing *Brassica napus* KAS factor A and *Ricinus communis* KAS factor A cDNA clones was used to obtain *C. hookeriana* KAS factor A genes. For KASIII, a spinach KASIII cDNA clone obtained from Dr. Jan Jaworski was radiolabeled and used as a probe to isolate a KASIII clone from C. *hookeriana.* For KAS B and KAS A cloning from C. *pullcherrima, C. hookeriana* KAS B and KAS A genes chKAS B-2 and chKAS A-2-7 (see below) were radiolabeled and used as probes.

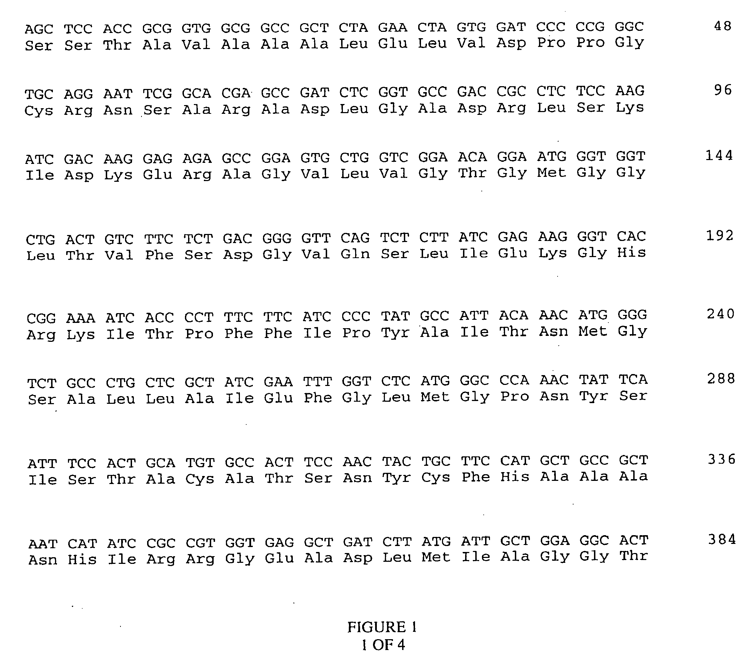

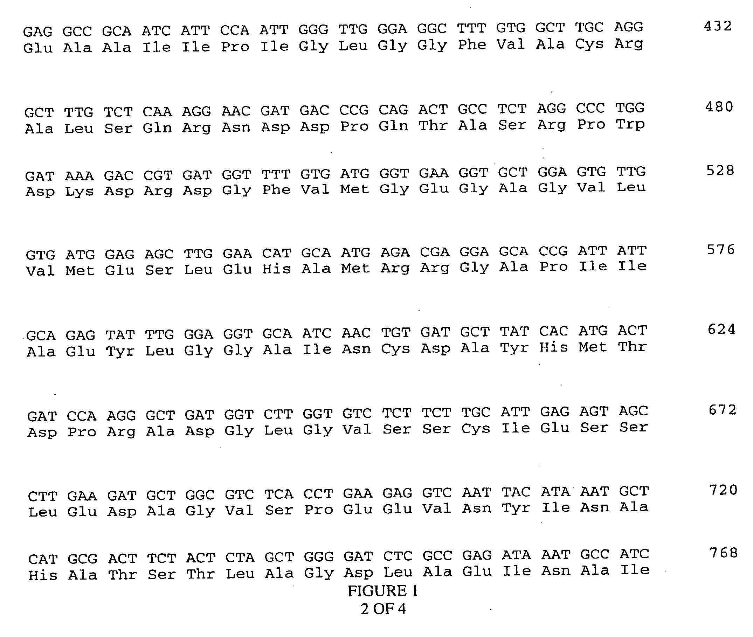

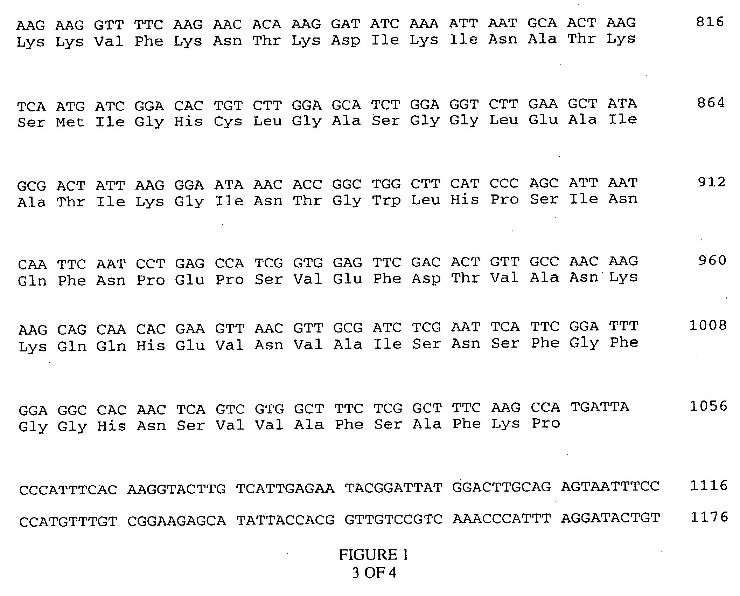

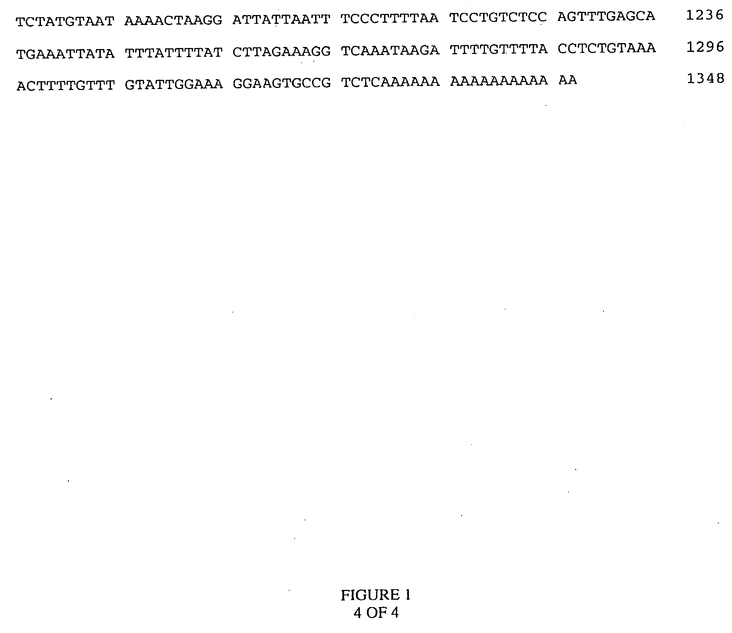

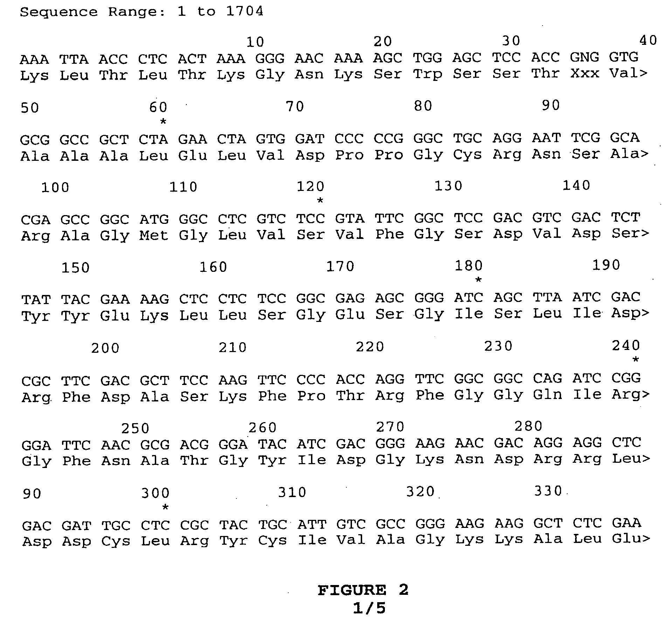

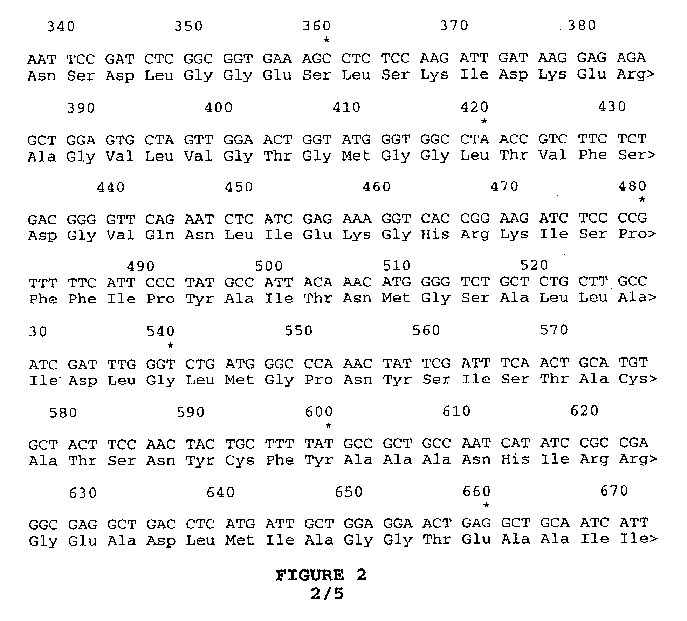

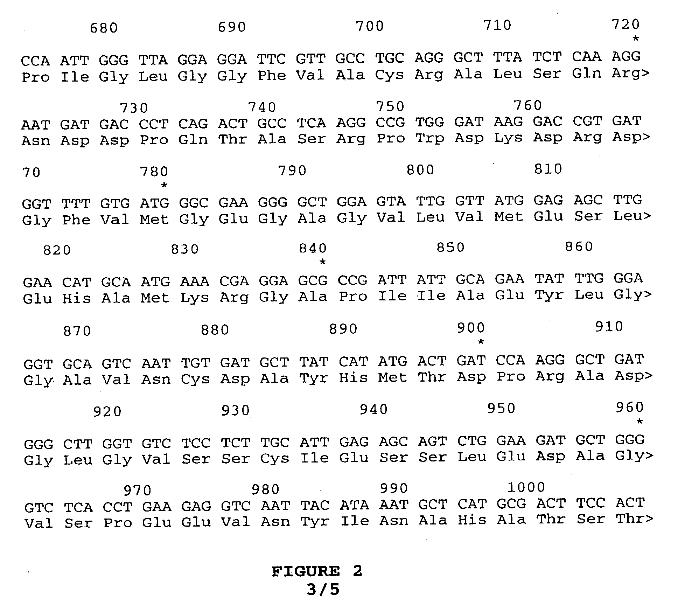

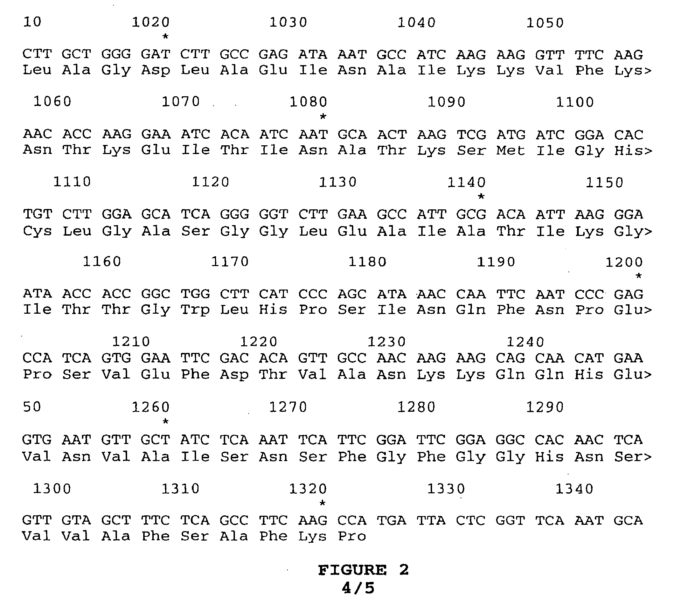

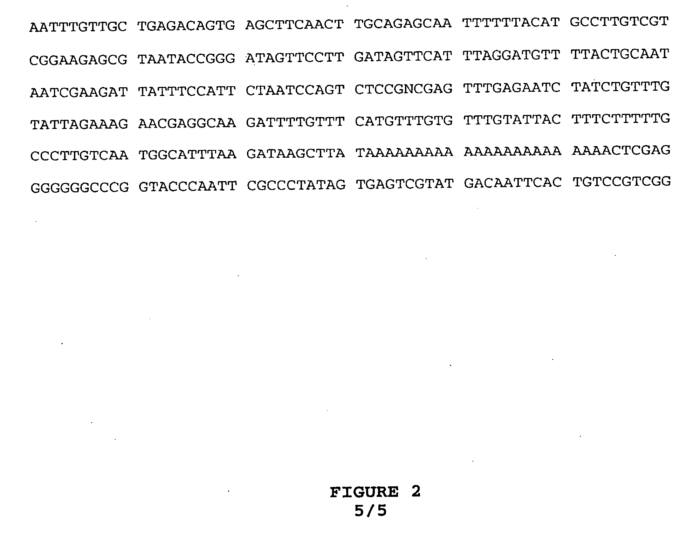

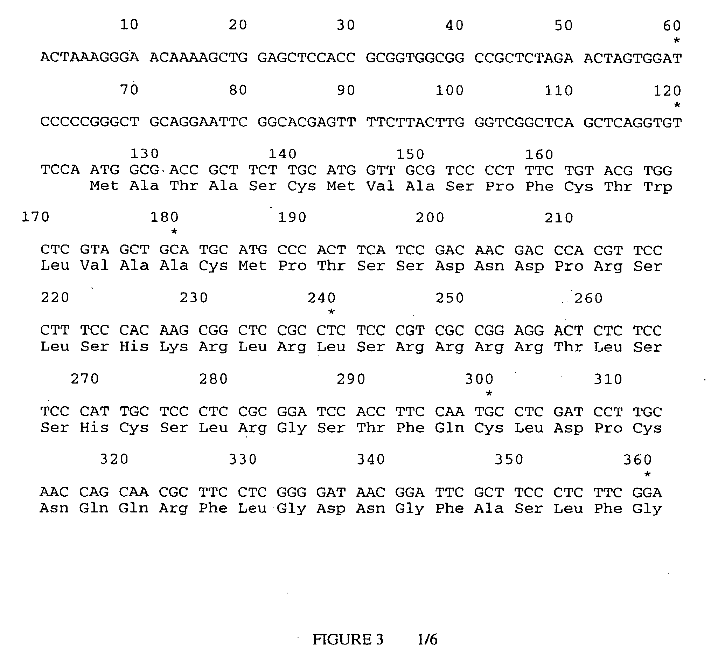

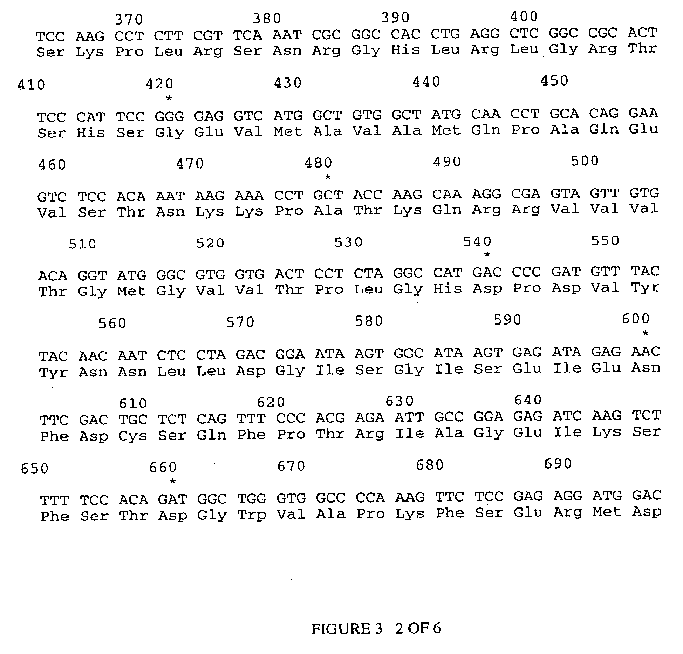

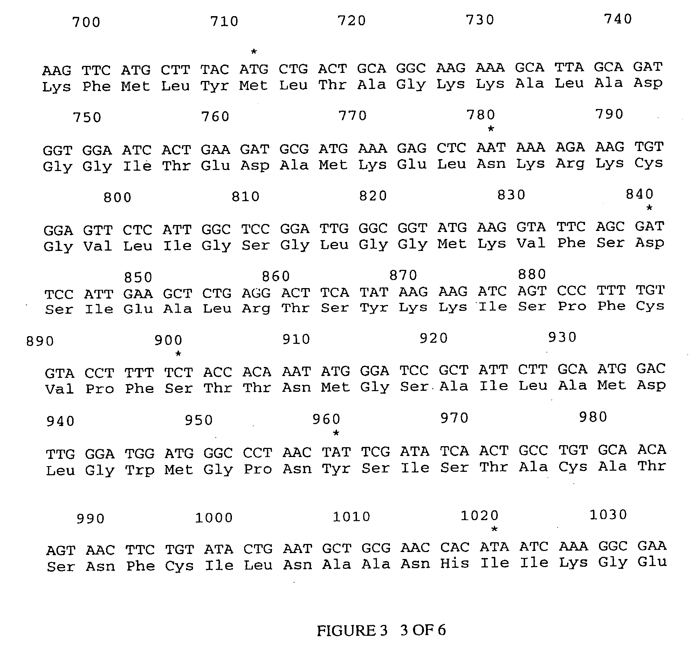

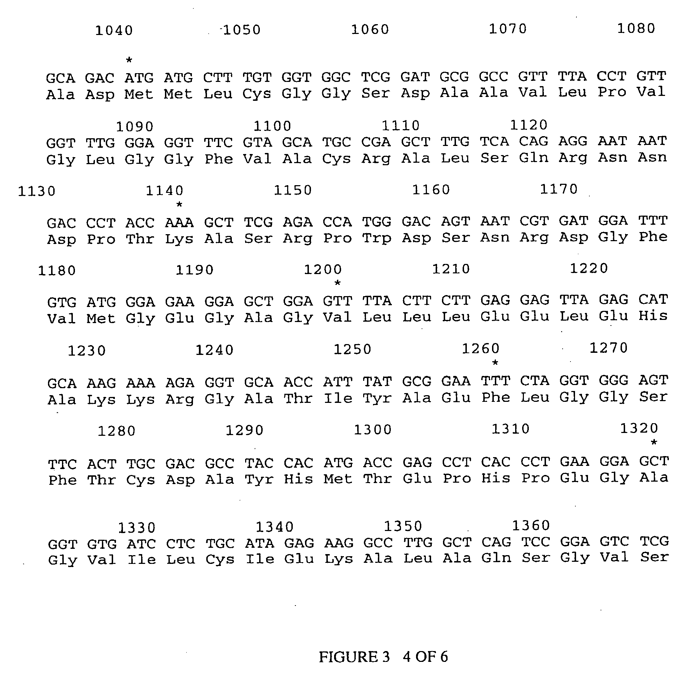

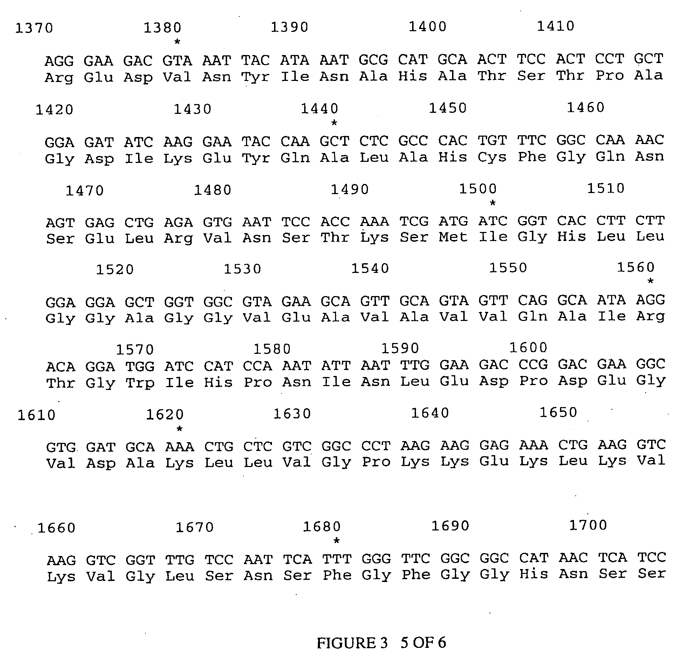

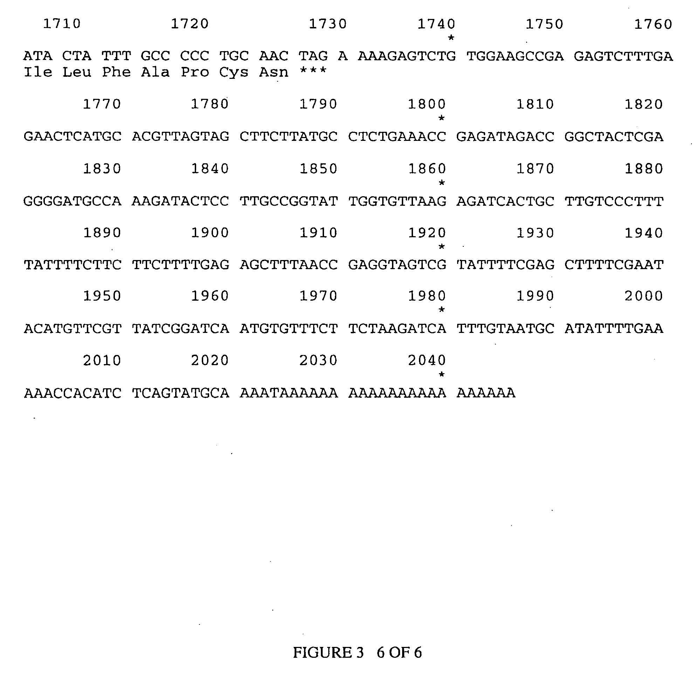

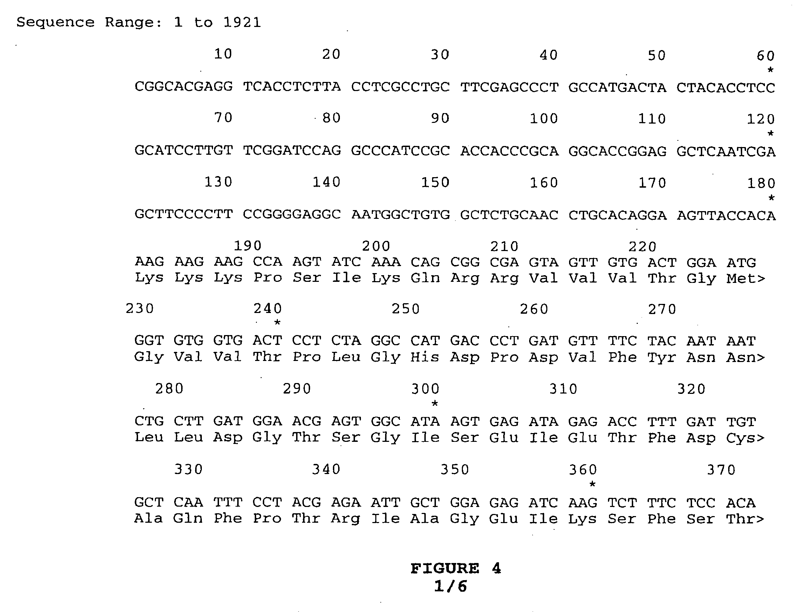

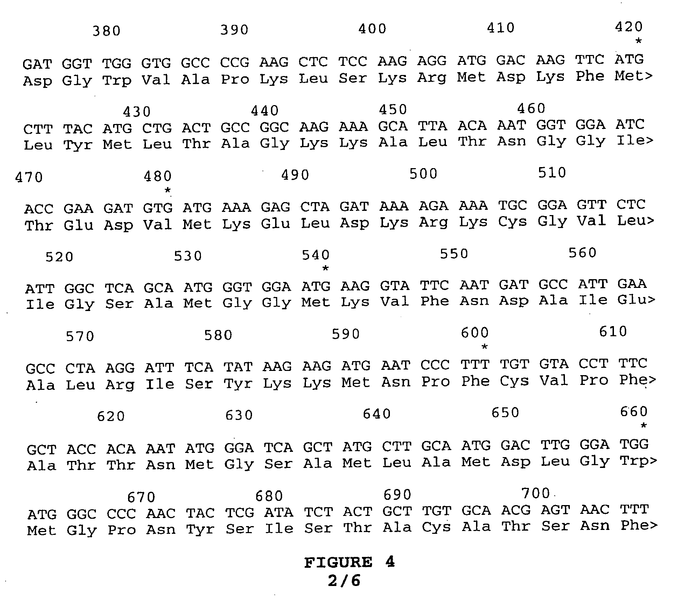

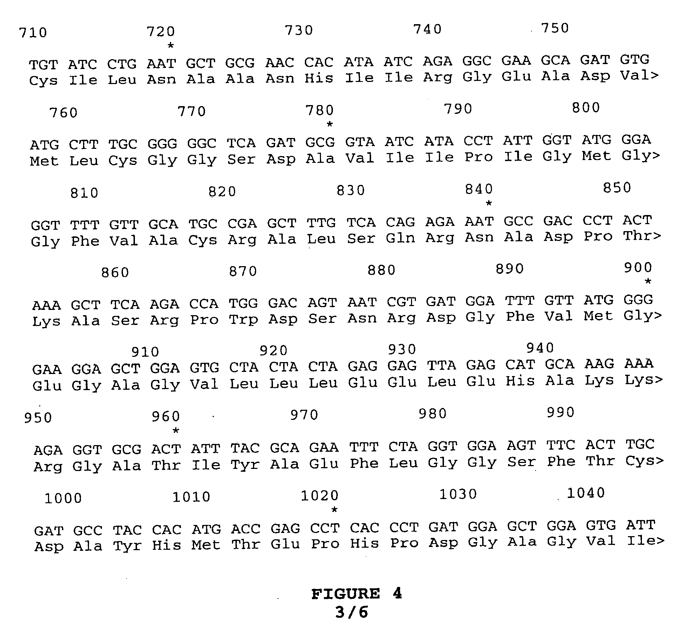

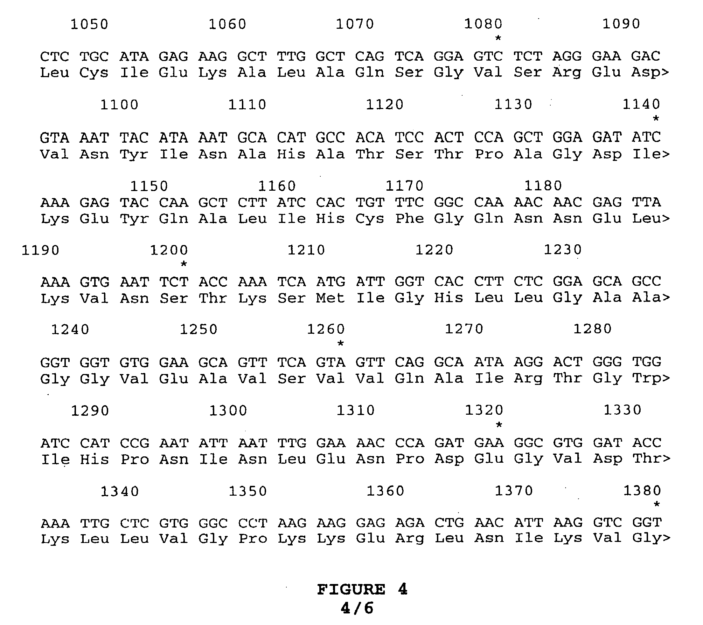

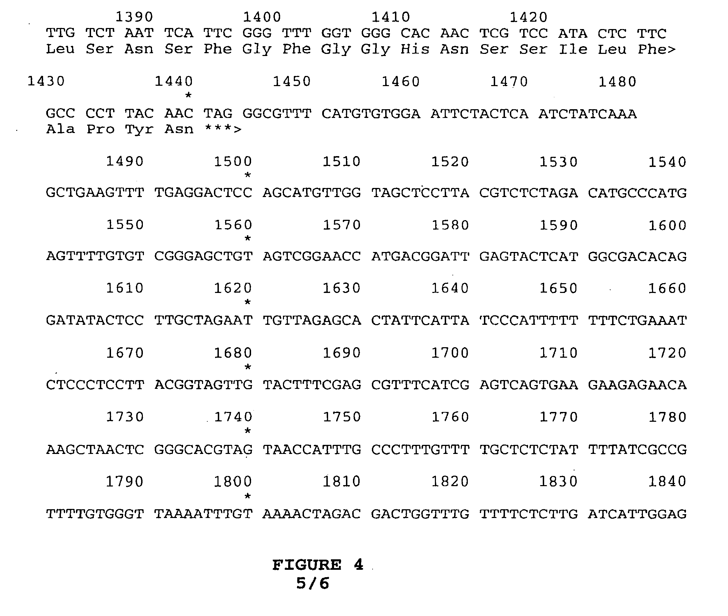

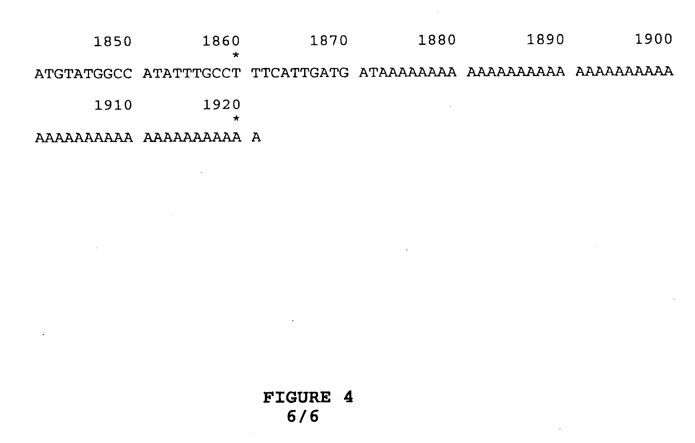

DNA sequence and translated amino acid sequence for *Cuphea* KAS clones are provided in Figures 1-9. *Cuphea hookeriana* KAS factor B clones chKAS B-2 and chKAS B-31-7 are provided in Figures 1 and 2.. Neither of the clones is full length *Cuphea hookexiana* KAS Factor A clones chKAS A-2-7 and chKAS A-1-6 are provided in Figures 3 and 4. chKAS A-2-7 contains the entire encoding sequence for the KAS factor protein. Based on comparison with other plant synthase proteins, the transit peptide is believed to be represented in the amino acids encoded by nucleotides 125-466. chKAS A-1-6 is not a full length clone although some transit peptide encoding sequence is present. Nucleotides 1-180 represent transit peptide encoding sequence, and the mature protein encoding sequence is believed to begin at nucleotide 181.

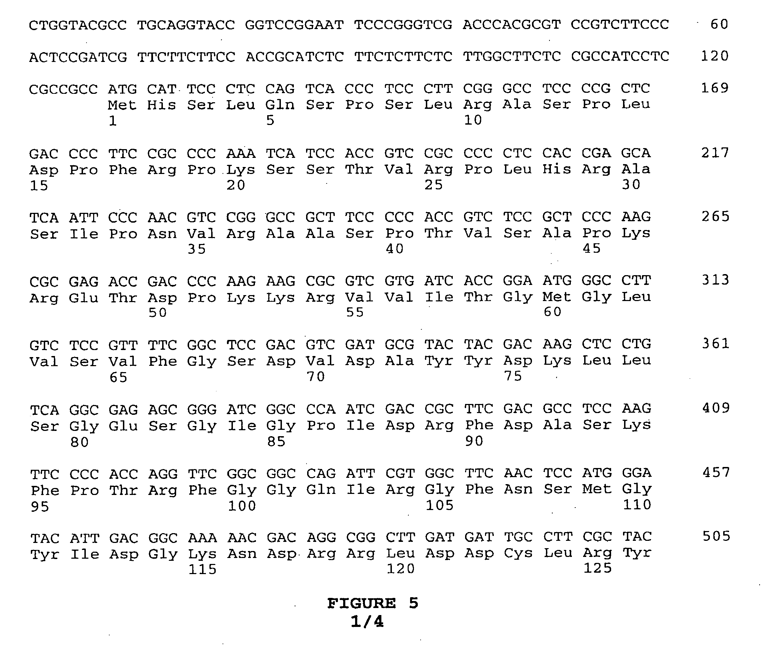

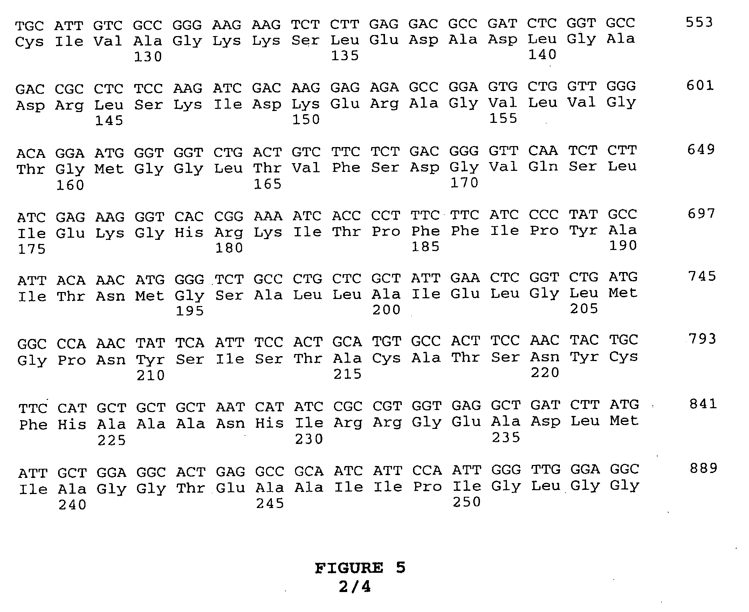

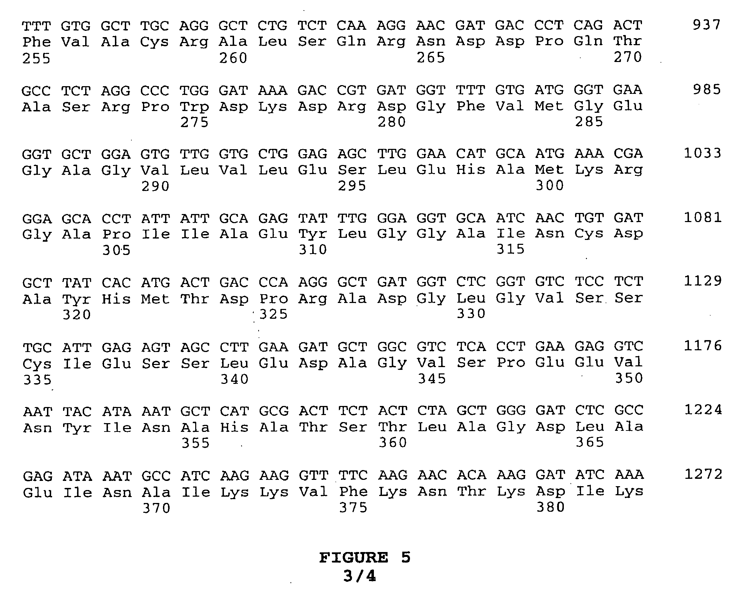

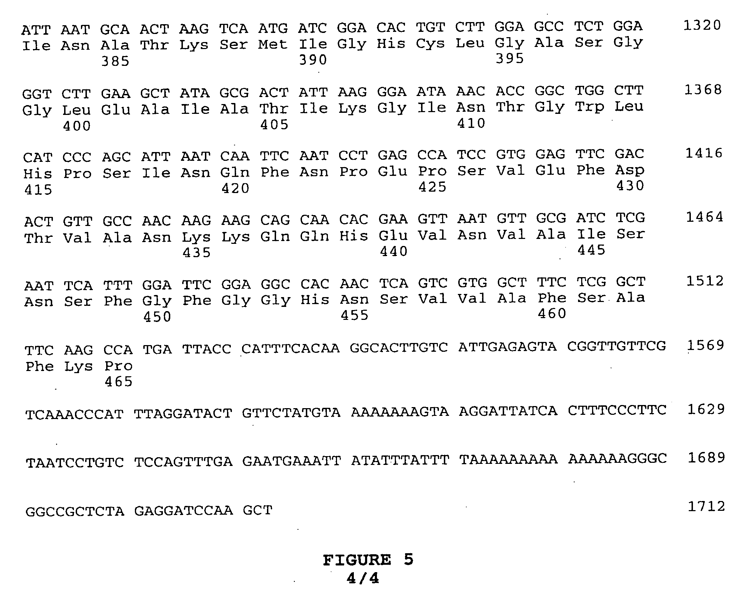

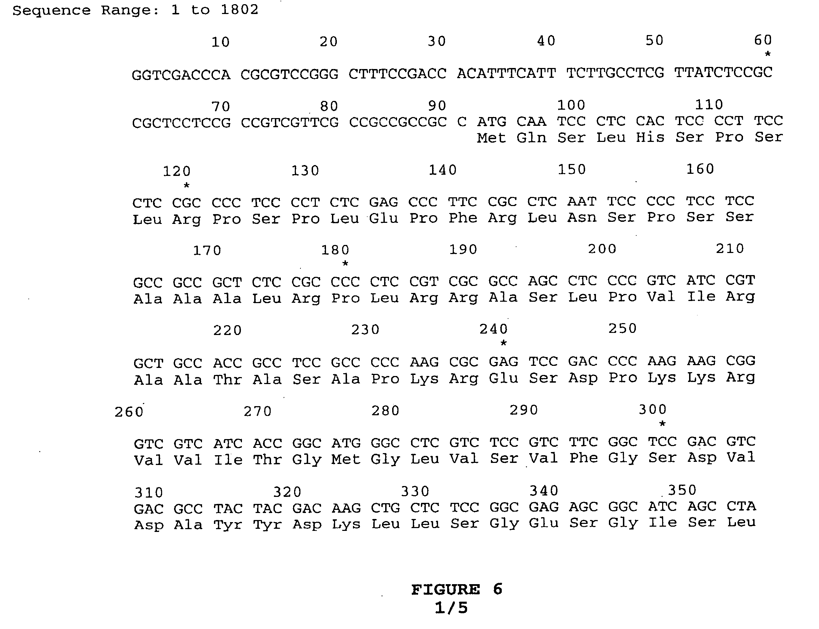

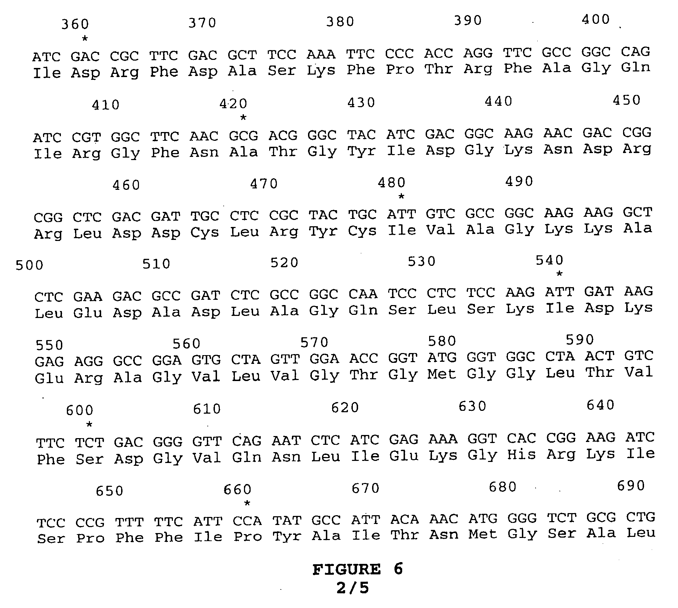

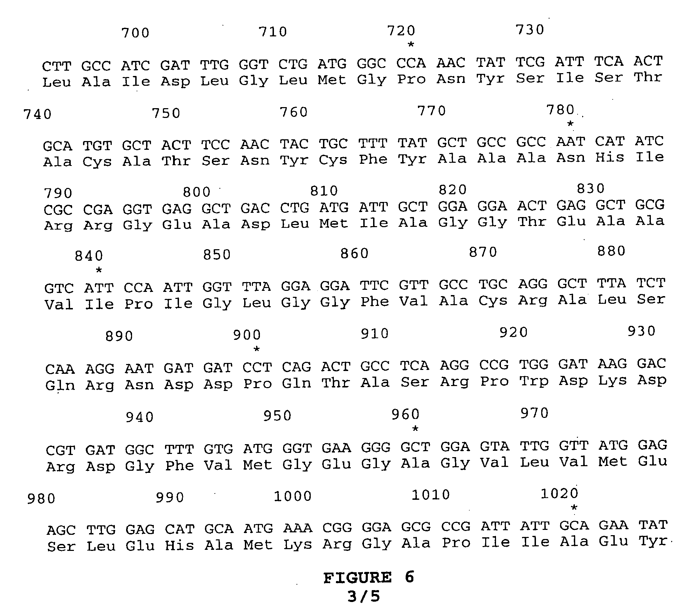

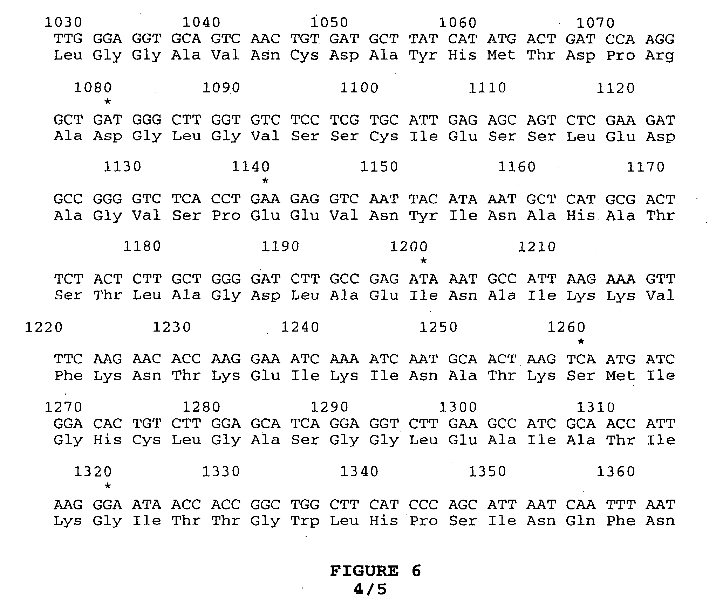

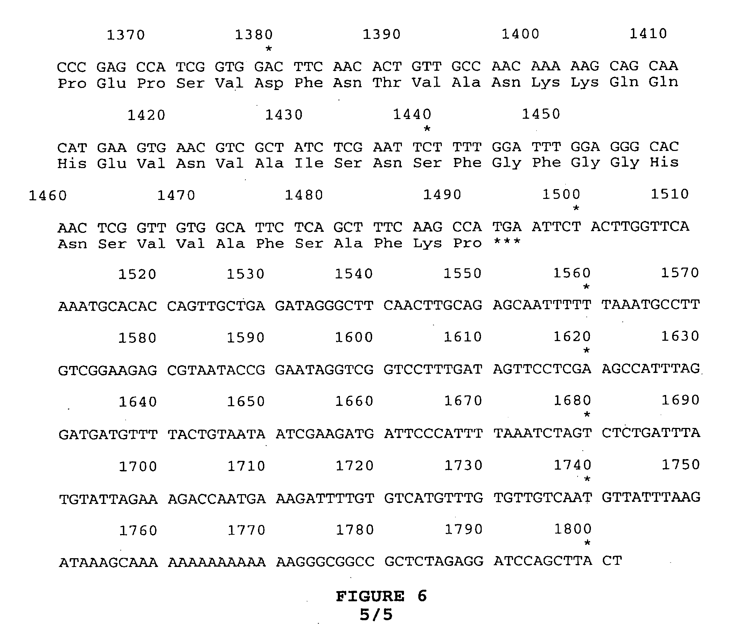

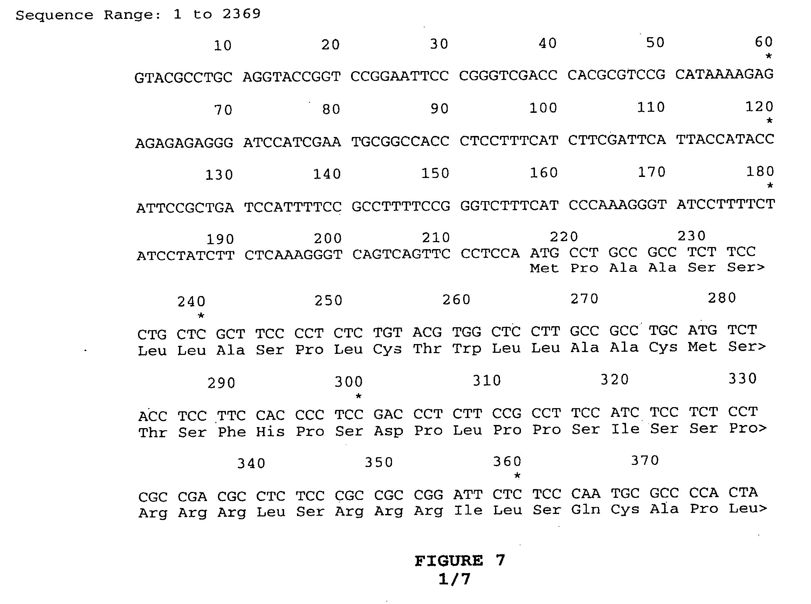

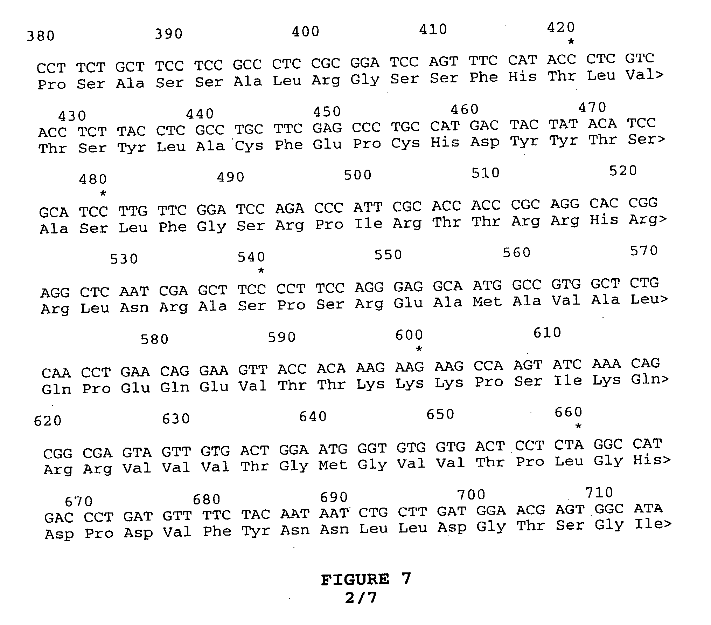

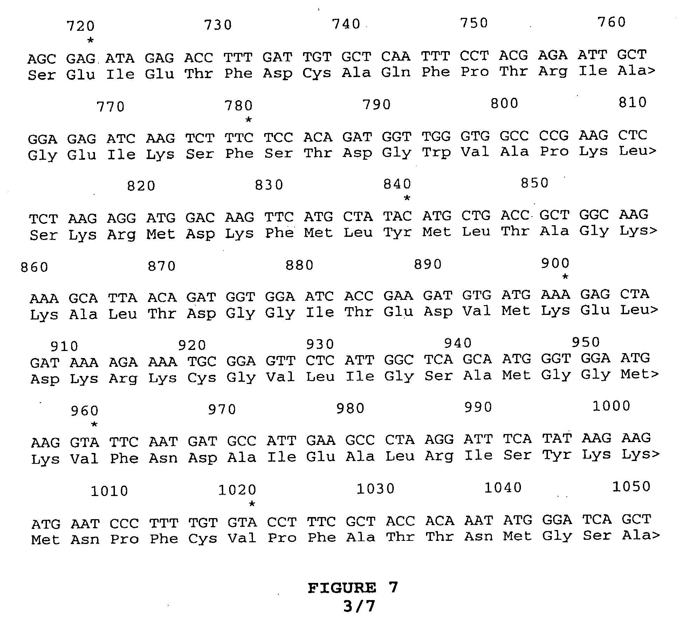

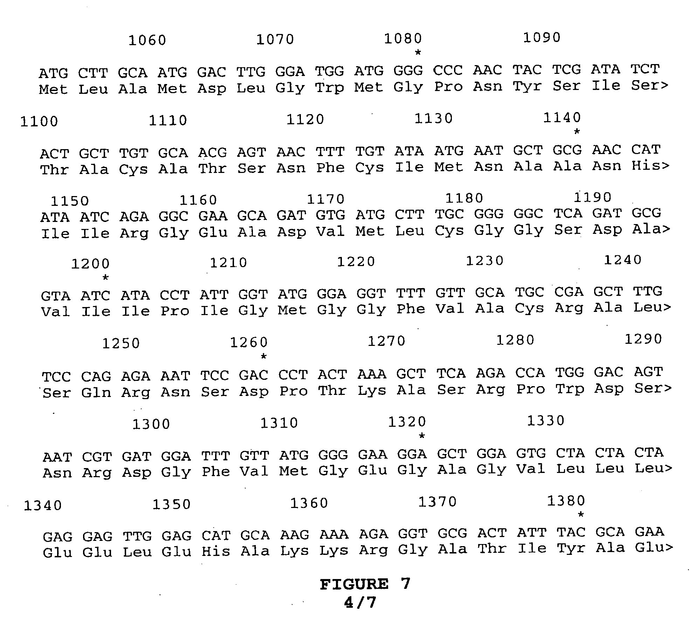

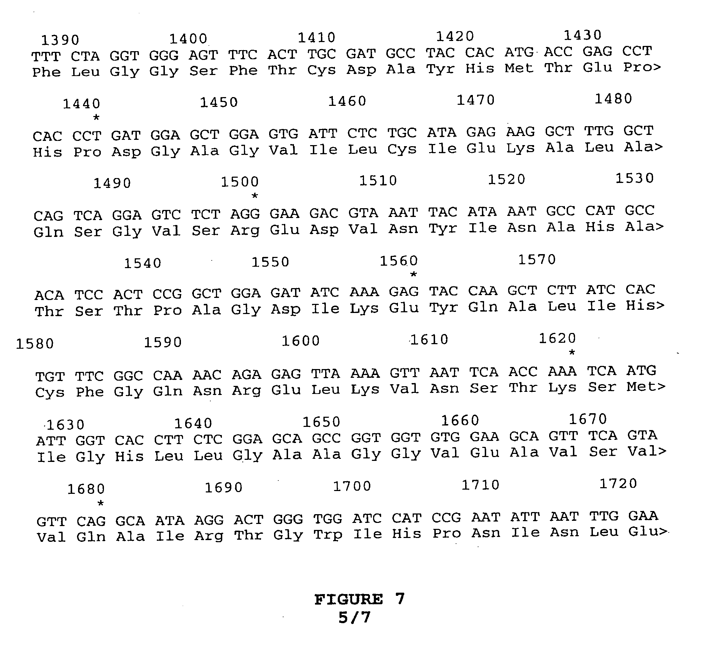

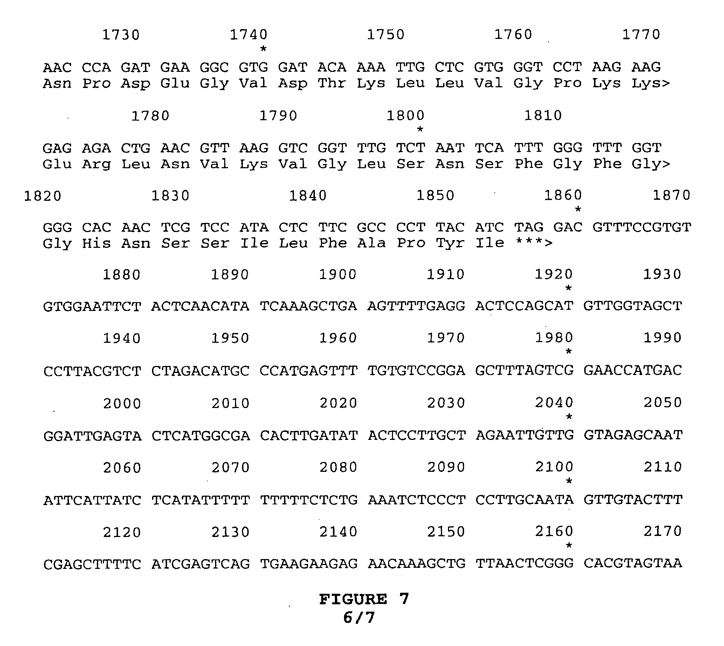

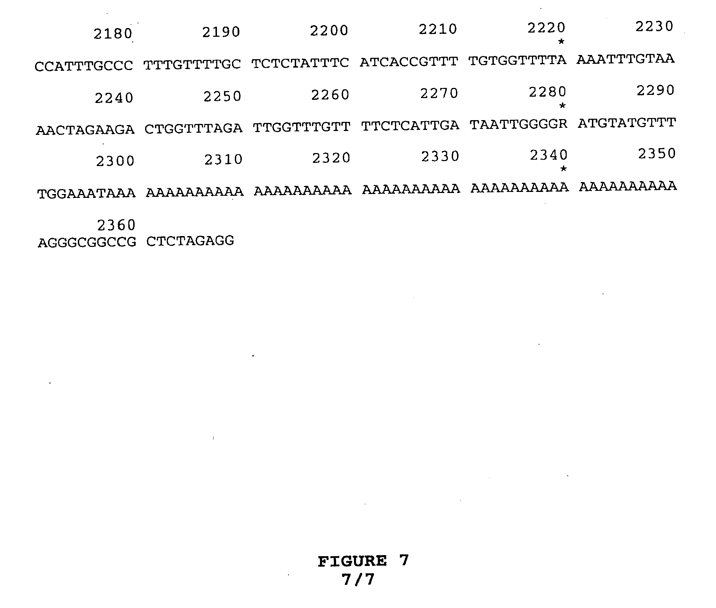

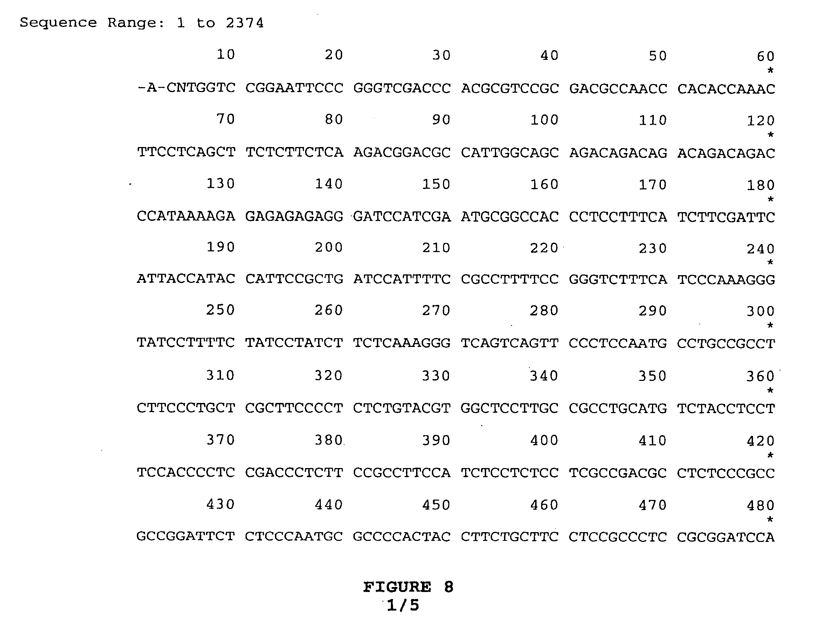

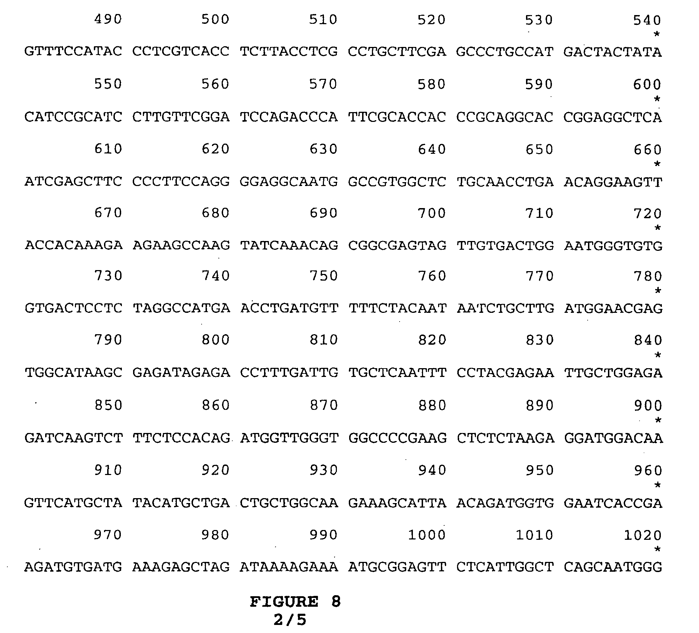

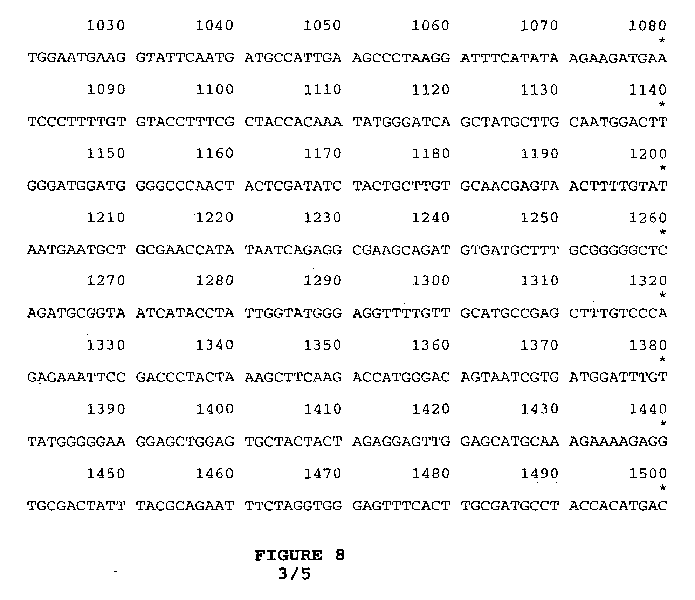

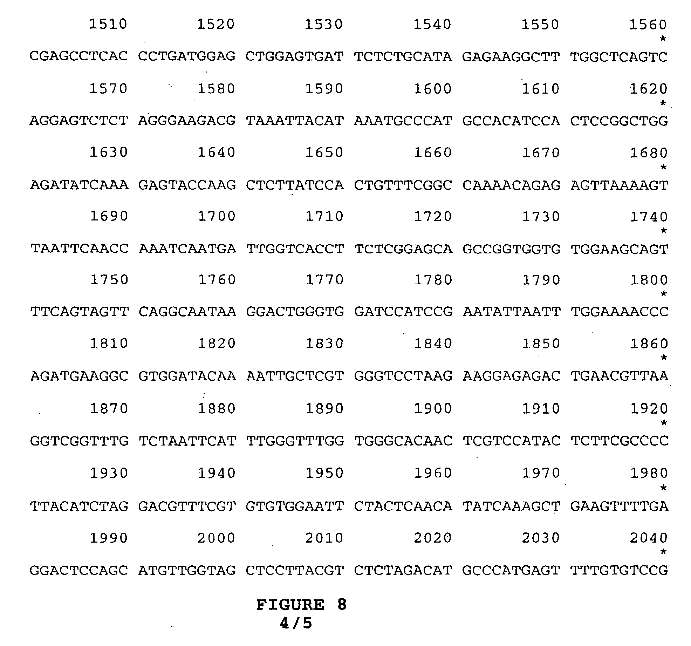

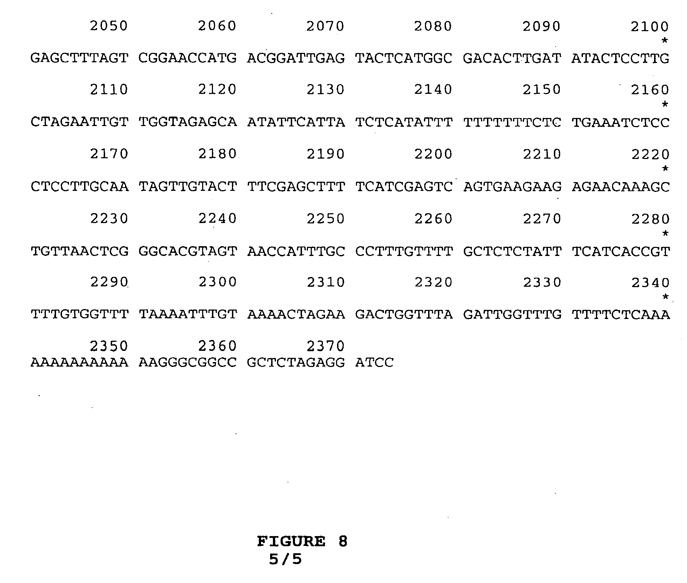

*Cuphea pullcherrima* KAS factor 13 clones cpuKAS B/7-8 and cpuKAS B/8-7A are provided in Figures 5 and 6. Both of the clones contain the entire encoding sequences for the KAS factor B proteins. The first 35 amino acids of cpuKAS B/7-8 are believed to represent the transit peptide, with the mature protein encoding sequence beginning at nucleotide 233. The first 39 amino acids of cpuKAS B/8-7A are believed to represent the transit peptide, with the mature protein encoding sequence beginning at nucleotide 209. *Cuphea pullcherrima* KAS factor A clones cpuKAS A/p7-6A and cpuKAS A-p8-9A are provided in Figures 7 and 8. Both of the clones contain the entire encoding sequences for the KAS factor A proteins. Translated amino acid sequence of cpuKAS A/p7-6A is provided. The mature protein is believed to begin at the lysine residue encoded 595-597, and the first 126 amino acids are believed to represent the transit peptide. The DNA sequence of KAS A clone cpuKAS A-p8-9A is preliminary. Further analysis will be conducted to determine final DNA sequence and reveal the amino acid sequence encoded by this gene.

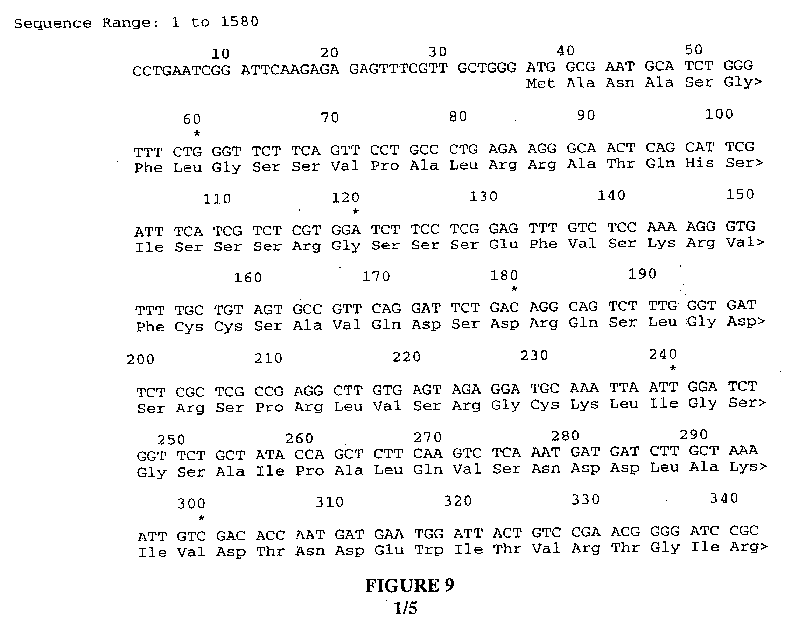

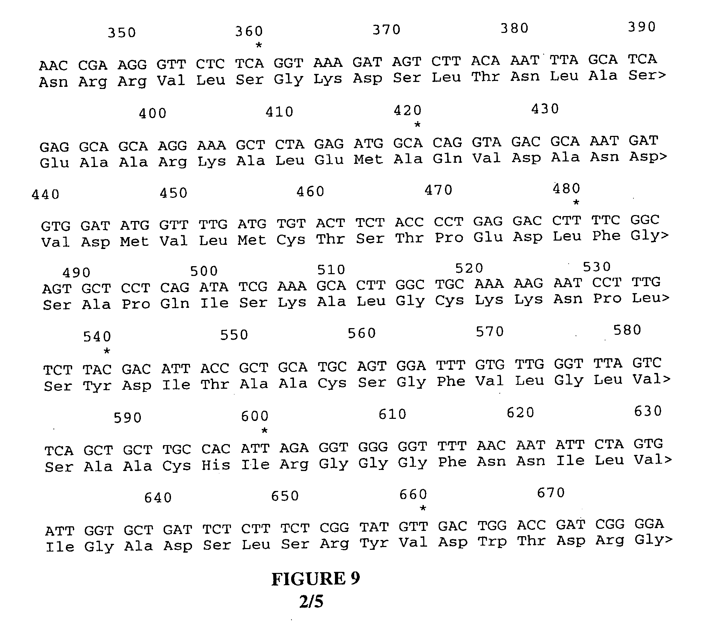

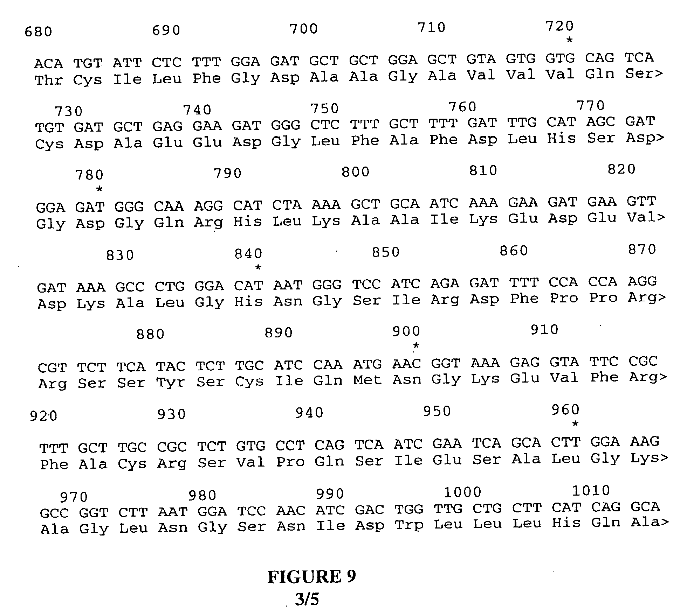

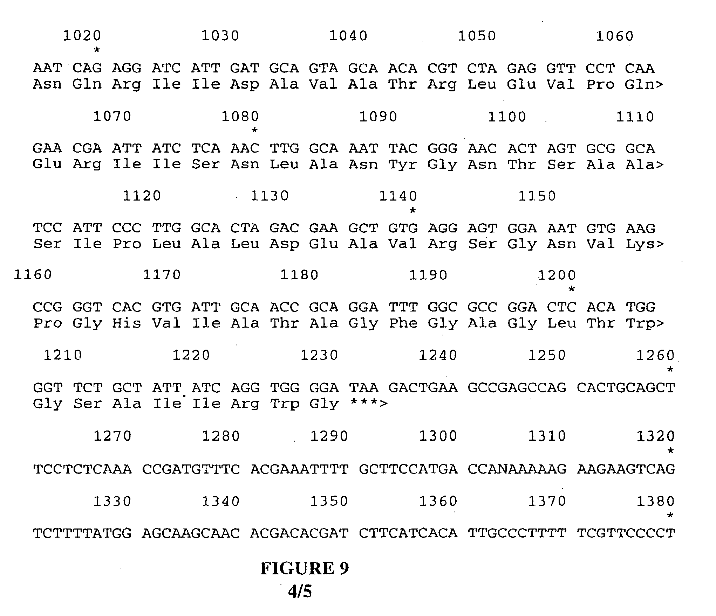

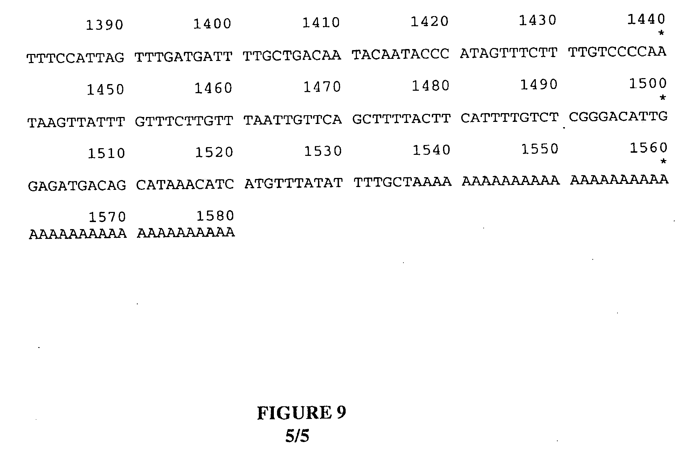

DNA and translated amino acid sequence of *Cuphea hookeriana* KASIII clone chKASIII-27 is provided in Figure 9. The encoding sequence from nucleotides 37-144 of chKASIII-27 are believed to encode a transit peptide, and the presumed mature protein encoding sequence is from nucleotides 145-1233.

Deduced amino acid sequence of the *C. hookeriana* KAS factor B and KAS factor A cDNA's reveals strong homology to the *Brassica napus* and *Ricinus communis* clones previously reported. The *C. hookeriana* KAS factor B clone is more homologous to the *Ricinus* and *Brassica* KAS factor B clones (94% and 91% respectively) than it is to the *Ricinus* and *Brassica* KAS factor A clones (60% for both). Furthermore, the *C. hookeriana* KAS factor A clone is more homologous to the Ricinus and *Brassica* KAS factor A clones (85% and 82% respectively) than it is the *Ricinus* and *Brassica* KAS factor B clone (60% for both). The *C. hookeriana* KAS factor B cDNAs designated as chKAS B-2 and chKAS B-31-7 are 96% identical within the mature portion of the polypeptide. Similarly, the deduced amino acid sequence of the mature protein regions of the *C. hookeriana* KAS factor A clones chKAS A-2-7 and chKAS A-1-6 are 96% identical. The *C. pullcherrima* KAS clones also demonstrate homology to the *R.* communes and *Brassica napus* KAS clones. The mature protein portion of all of the KAS factor A family members i.n the different *Cuphea* species are 95% identical. Similarly the mature protein portion of the KAS factor B genes in Cuphea are also 95-97% identical with each other. However there is only approximately 60% sequence identity between KAS factor B and KAS factor A clones either within the same or different species of *Cuphea.*

### Example 2 Levels and Patterns of Expression

To examine tissue specificity of KAS expression in *Cuphea hookeriana,* Northern blot analysis was conducted using total RNA isolated from seed, root, leaf and flower tissue. Two separate but identical blots were hybridized with either chKAS B-31-7 or chKAS A-2-7 coding region probes. The data from this RNA blot analysis indicate that KAS B is expressed at a similar level in all tissues examined, whereas KAS A expression is detected only in the seed. These results also demonstrate a different level of expression for each of the synthases. KAS A is an abundant message, whereas KAS B is expressed at low levels. Furthermore, even under highly stringent hybridizati.on conditions (65_C, 0.1 X SSC, 0.5% SDS), the KAS A probe hybridizes equally well with two seed transcripts of 2.3 and 1.9 kb. The larger hybridizing band is likely the transcript of the KAS A-2-7 gene since the size of its cDNA is 2046bp, and the number of clones obtained from cDNA screening corresponds well with the apparent mobility of the mRNA and its abundance on the blot.

### Example 3 Expression of Plant KAS Genes in E.coli

DNA fragments encoding the mature polypeptide of the *Cuphea hookeriana* KAS A cDNAs and the *Cuphea pullcherrima* KAS B cDNAs were obtained by PCR and cloned into a QIAexpress expression vector (Qiagene). Experimental conditions for maximum level of expression were determined for all of these clones and the parameters for highest level of soluble fraction were identified. Cells are grown in ECLB media containing 1M sorbitol and 2.5 mM betaine overnight and subcultured as a 1:4 dilution in the same medium. Cells are then grown for 2 hours (to approximately .6-.8 O.D.) and induced with 0.4 mM IPTG and allowed to grow for 5 more hours.

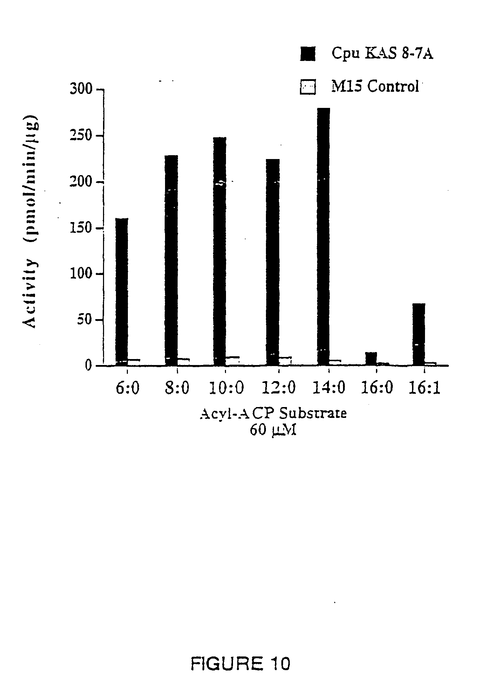

Enzyme activity of the affinity purified recombinant enzymes obtained from over-expression of the chKAS A-2-7 and cpuKAS B/8-7A clones was measured using a wide range of acyl-ACP substrates (6:0- to 16:1-ACP). The activity profile for cpuKAS B/8-7A is provided in Fig.10. The data demonstrate that the enzyme is active with all acyl-ACP substrates examined, although activity on 6:0 to 14:0-ACP substrates is substantially greater than the activity on 16:0 and 16:1 substrates.

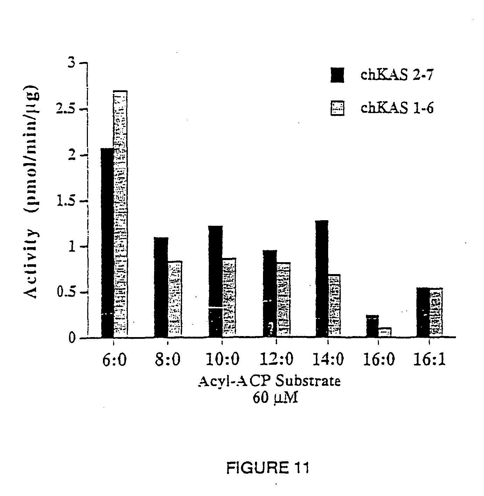

The activity profile of the *C*. *hookeriana* KAS A clones chKAS A-2-7 and chKAS A-1-6 is provided in Figure 11. The C. *hookeriana* KAS A clones are most active with C:6, and have the least activity with C:16:0 substrates. However, the activity of this clone on even the preferred C6:0 substrate is 50 fold lower than the activity of the *C. pullcherrima* KAS B clones.

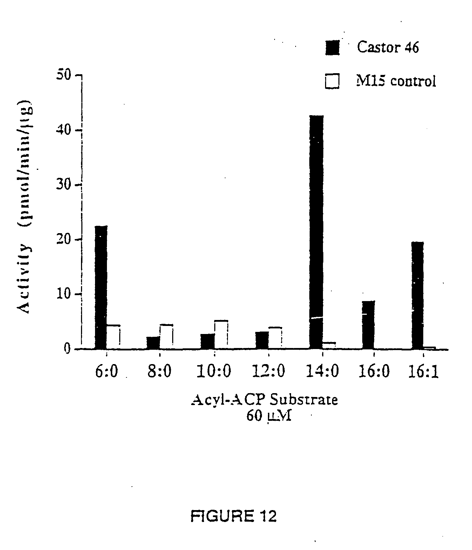

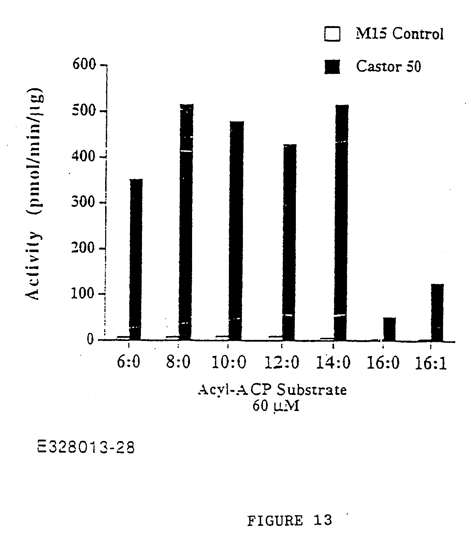

A fragment containing the mature protein encoding portion of a *R. communis* KAS factor A clone was also cloned into a QIAexpress expression vector, expressed in *E. coli* and the enzyme affinity purified as described above. The activity profile for castor KAS A is provided in Figure 12. Highest activity is observed with C14:0 substrates, although some activity is also seen with C6:0 and C16:1. In comparison, the activity profile obtained from purified *R. communis* KAS factor B also using the QIAexpress expression system is provided in Figure 13. The KAS B clone demonstrates substantially higher levels of activity (10 fold and higher) than the *R. communis* KAS A clone. The preference of the KAS factor B for 6:0- to 14:0-ACP substrates is consistent with the previous observations that this protein provides KAS I activity.

### Example 4 KAS and TE Expression in Transgenic Seed

Both the CpFatB1 *(C. hookeriana* thioesterase cDNA; Dehesh *et al.* (1996) *Plant Physiol. 110*:203-210) and the chKAS A-2-7 were PCR amplified, sequenced, and cloned into a napin expression cassette. The napin/cp FatB1 and the napin/KAS A-2-7 fusions were ligated separately into the binary vector pCGN1558 (McBride and Summerfelt (*Pl. Mol. Biol.* (1990) *14*.269-276) and transformed into *A. tumefaciens,* EHA101. The resulting CpFatB1 binary construct is pCGN5400 and the chFCAS A-2-7 construct is pCGN5401. *Agrobacterium* mediated transformation of a *Brassica napus* canola variety was carried out as described by Radke *et al*. (*Theor*. *Appl. Genet*. (1988) 75:685-694; *Plant Cell Reports* (1992) *11*:499-505). Several transgenic events were produced for each of the pCGN5400 and pCGN5401 constructs.

A double gene construct containing a napin/cpFatB1 expression construct in combination with a napin/chKAS A-2-7 expression construct was also assembled, ligated into a binary vector and used for co-cultivation of a canola *Brassica* variety. The binary construct containing the chFatB1 and chKAS A-2-7 expression constructs is pCGN5413.

Fatty acid analysis of 26 transgenic lines containing chKAS A-2-7 (5401 lines) showed no significant changes in the oil content or profile as compared to similar analyses of wild type canola seeds of the transformed variety.

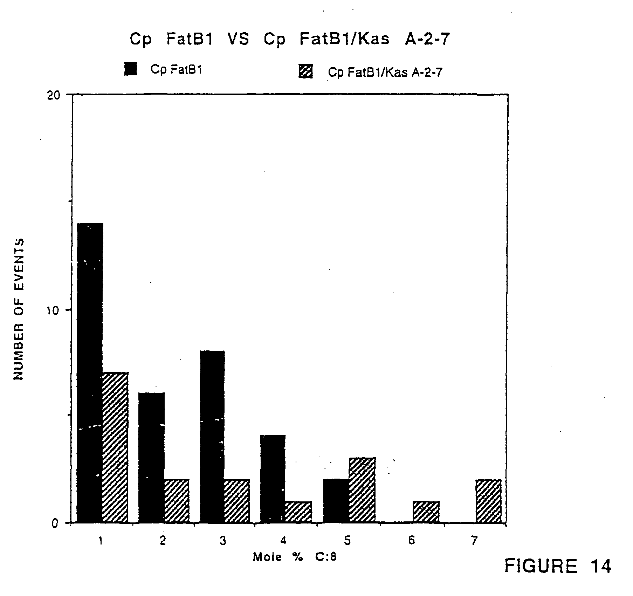

Fatty acid analysis of 36 transgenic lines containing cpFatB1 (5400 lines) showed increased levels of C:8 and C:10 in transgenic seeds. The highest level of C:8 observed in a pool seed sample was 4.2 mol% The C:10 levels were between 30 and 35% of the C:8 content. Fatty acid analysis of 25 transgenic lines contai.ning the TE/KAS A tandem (5413 lines) demonstrated an overall increase in both C:8 and C:10 levels relative to those observed with TE containing lines (5400) alone. In lines containing the cpFatB1 construct alone, the average level of C:8 average were 1.5 mol%, whereas the C:8 average levels in TE/KAS A tandem containing lines was 2.37 mol%. The ratio of C:8 to C:10 remained constant in both populations. The number of transgenic events relative to the C:8 content are presented in Figure 14. These data show that the transgenic events with tandem TE/KAS A construct yield more lines with higher levels of C:8 than those events with single TE construct. For example, several lines containing nearly 7 mole% C8 were obtained with the TE/KAS A pCGN5413 construct, whereas the highest C8 containing line from the pCGN5400 TE alone transformation contained 4.2 mole% C8.

Half seed analysis of the T3 generation of transgenic canola plants expressing a ChFatB2 (*C. hookeriana* thioesterase; Dehesh *et al.* (1996) *The Plant Journal 9*:167-172) indicate that these plant can accumulate up to 22 weight% (33 mol%) of 8:0 and 1.0:0 fatty acids (4804-22-357). Segregation analysis shows that these transformants contain two loci and that they are now homozygous. Selected plants grown from these half seeds were transferred into the greenhouse and later crossed with T1 transformants that had been transformed with either *Cuphea hookeriana* KAS A (5401) alone or KAS A/CpFatB1 double constructs (5413)

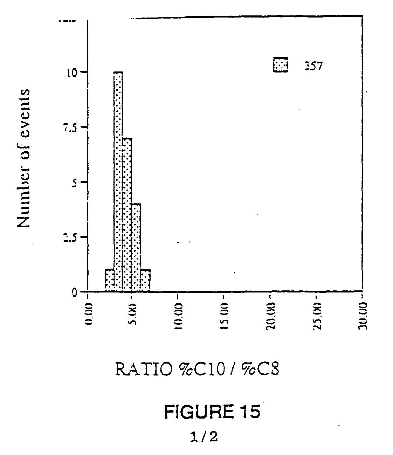

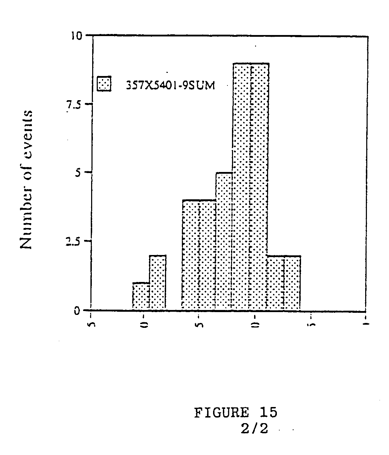

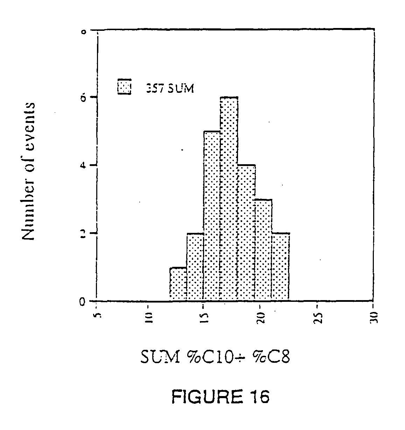

Fatty acid analysis of several events resulting from the crosses between transgenic lines containing ChFatB2 (4804-22-357) and chKAS A-2-7 (5401-9), reveal an increase in the ratio of C:10/C:8 levels (Figure 15). This C:10/C:8 ratio in nearly all of the transgenic events containing ChFatB2 TE alone fluctuates between 3 and 6, whereas in the F1 generation of transgenic containing both the TE and the KAS A-2-7, the ratio can be as high as 22. This increase in C:10 levels is accompanied by an increase in the total C:8 and C:10 content (Figure 16). The sum of the C:8 and C:10 fatty acids in the heterozygous F1 lines is as high as those in the homozygous parent line (4804-22-357), whereas the heterozygous lines usually contain substantially less C:8 and C:10 than the homozygous lines.

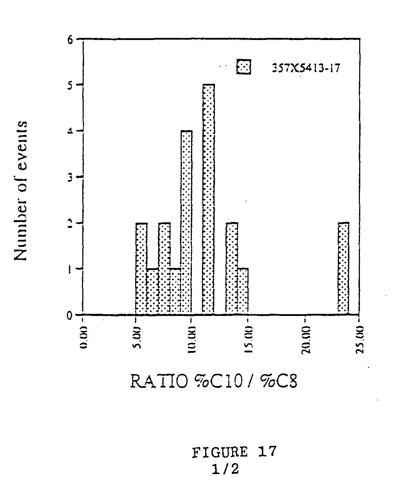

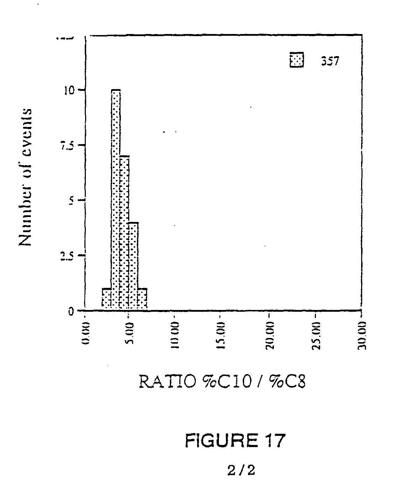

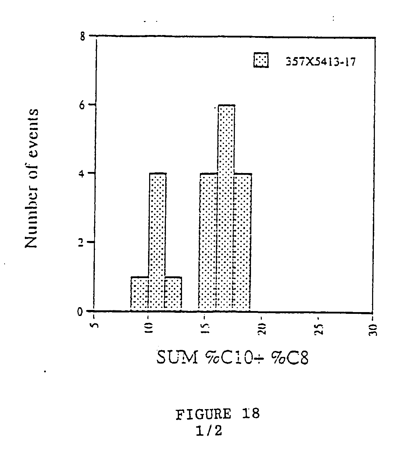

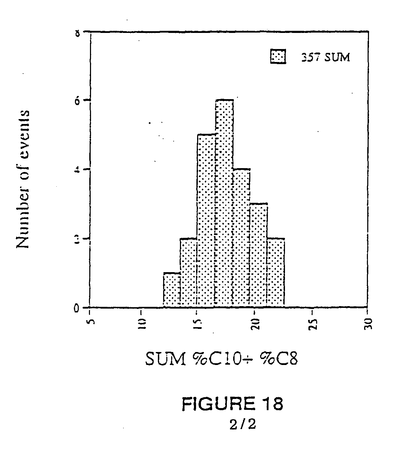

Similar results were observed in F1 generation seeds resulting from crosses performed between 4804-22-357 (ChFatB2) and the 5413-17 event (CpFatB1 and chKAS A-2-7 tandem). Levels of C:8 and C:10 in the 5413-17 line were 6.3 and 2.8 mol% respectively. Data presented in Figure 17 show that there is shift towards C:10 fatty acids as was observed with the 4804-22-357 (ChFatB2) x 5401-9 (chKAS A-2-7) crosses. Furthermore, Figure 18 indicates the presence of two separate populations of heterozygotes. Those containing approximately 9-11 weight percent C:10 + C:8 are believed to represent offspring containing a single copy of the ChFatB1 TE gene and no copies of the CpFatB1 and chKAS A genes from 5413. Those plants containing approximately 15-20 weight percent C:10 + C:8 are believed to represent the heterozygotes containing a single ChFatB1 TE gene as well as the CpFatB1 and chKAS A genes from 5413. Thus, the level of the C:10 + C:8 fatty acids does not decrease to 50% of that detected in parent lines when a copy of the ChKAS A gene is present.

To further characterize the chain length specificity of the *Cuphea hookeriana* KAS A enzyme, crosses between transgenic *Brassica napus* lines containing a California Bay (*Umbellularia californica*) 12:0 specific thioesterase, Uc FatB1 (USPN 5,344,771) and chKAS A-2-7 (5401-9) were made. Half seed analysis of transgenic plants containing Uc fatB1 have previuosly indicated that these plants can accumulate up to 52 mol% C12:0 in the seed oil of homozygous dihaploid lines (LA86DH186). Crosses between the line LA86DH186 and untransformed control *Brassica* demonstrated a decrease in the C12:0 levels.

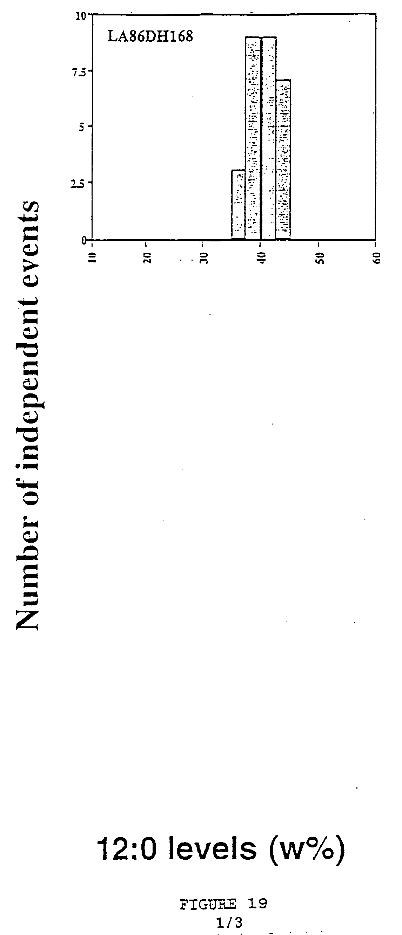

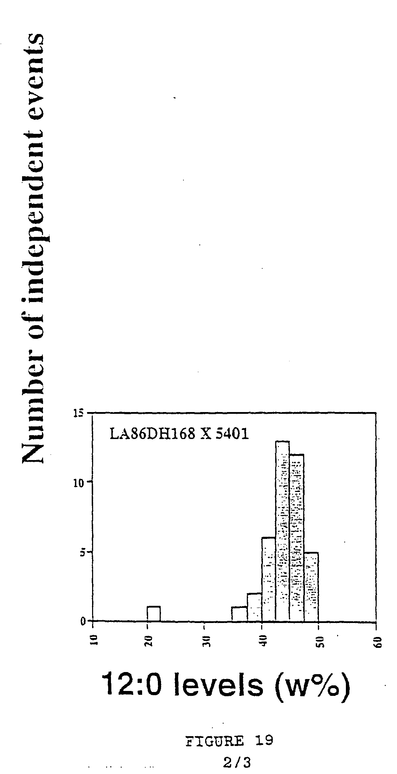

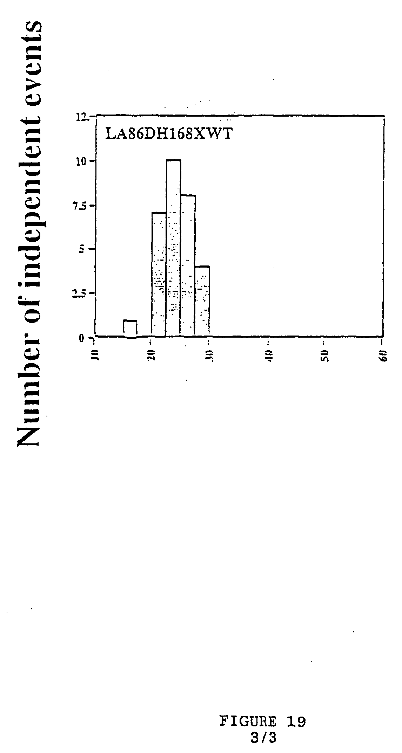

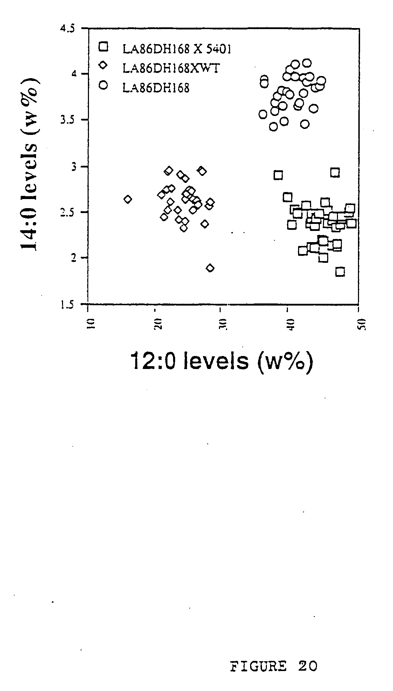

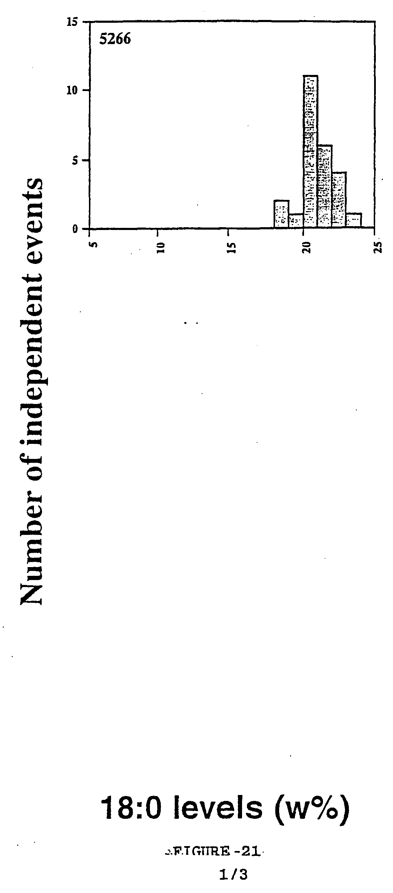

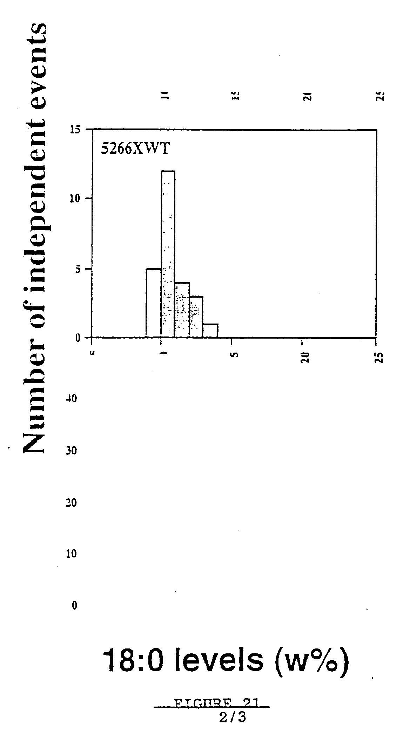

However, crosses between LA86DH186 and the 5401-9 hemizygous line led to an accumulation of up to 57 mol% C12:0 in the seed oil of F1 progeny (Figure 19). Interestingly, in crosses with LA86DH186 x untransformed control line and LA86DH186 x 5401-9, levels of C14:0 in the seeds of the F1 progeny decreased to 50% of the levels obtained in homozygous LA86DH186 lines (Figure 20). Furthermore, increases in the proportion of C12:0 fatty acid resulted in a substantial decline in the proportions of all the long-chain fatty acyl groups (C16:0, C18:0, C18:2, and C18:3). These results indicate that the ChKAS A-2-7 is an enzyme with substrate specificity ranging from C6:0 to C10:0-ACP, and that its over-expression ultimately reduces the longer chain acyl-ACP pools.

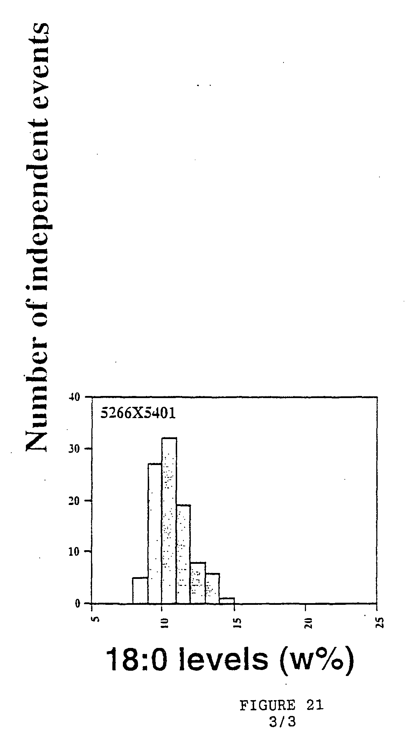

Further evidence is obtained in support of the chain length specificity of the ChKAS A-2-7 in crosses of the 5401-9 line with a transgenic line (5266) expressing an 18:1/18:0 TE from *Garcinia mangostana* (GarmFatA1, US patent appl.ication No. 08/440,845). Transgenic *Brassica* line 5266 has been shown to accumulate up to 24 mol% C18:0 in the seed oil of homozygous lines (Figure 21). However, in the seed oil of F1 progeny of crosses between 5266 and 5401-9 levels of C18:0 were reduced to approximately 12 mol%. Furthermore, levels of C16:0 generated from these crosses was similar to the levels obtained from the seed oil of nontransgenic control plants.

### Example 5 In vitro Analysis of Plant KAS Enzymes

Seed extracts were prepared from developing seeds of nontransgenic controls or transgenic *Brassica* expressing chKAS A-2-7 as described in Slabaugh *et al.* (*Plant Journal*, 1998 in press) and Leonard *et al.* (Plant Journal, 1998, in press). *In vitro* fatty acid synthesis assays were performed as described by Post-Beittenmiller (*J. Biol. Chem.* (1991), 266:1858-1865). Extracts were concentrated by ammonium sulfate precipitation and desalting using P-6 columns (Bio-Rad, Hercules, CA). Reactions (65µl) contained 0.1M Tris/HCl (pH 8.0), 1 mM dithiothreitol, 25 mM recombinant spinach ACP1, 1 mM NADH, 2 mM NADPH, 50 µM malonyl-CoA, 10 µM [1-¹⁴C]acetyl-CoA (50 mCi/mmol), 1mg/ml BSA, and 0.25 mg/ml seed protein. Selected seed extracts were preincubated with cerulenin at 23°C for 10 min. Reaction products were separated on an 18% acrlamide gel containing 2.25M urea, electroblotted onto to nitrocellulose and quntitated by phosporimaging using Image QuaNT software (Molecular Dynamics, Sunnyvale, CA). Authentic acyl-ACPs were run in parallel, immunoblotted and finally detected by anti-ACP serum to confirm fatty acid chain lengths.

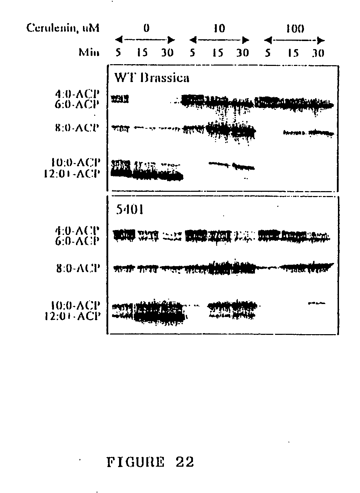

The results (Figure 22) indicate that the fatty acid synthesis capabilities of transgenic B.rasica (5401-9) seed extracts was greater than that obtained from in the nontransgenic controls as measured by the relative abundance of C8:0- and C10:0-ACP at all time points tested. In addition, pretreatment of the extracts with cerulenin, markedly reduced the synthesis of longer chain fatty acids in both the transgenic and nontransgenic control seed extracts. However, the extension of the spinach-ACP was much less inhibited in the seed extracts from the transgenic lines than in the seed extracts of nontransgenic control *Brassica.*

These data further support that Ch KAS A-2-7 is a condensing enzyme active on medium chain acyl-ACPs, and that expression of this enzyme in plants results in enlarged substrate pools to be hydrolyzed by medium-chain specific thioesterases. Furthermore, these data suggest that chKAS A-2-7 also is a cerulenin-resistant condensing enzyme.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claim.

A DNA construct comprising an encoding sequence for a KAS protein demonstrating activity preferential for medium chain fatty acid substrates of C6:0-ACP, C8:0-ACP and C10:0-ACP.

A DNA construct as defined above wherein said KAS protein is from a *Cuphea* species.

The above construct wherein said KAS protein is a KAS factor A protein.

The above construct wherein said KAS protein is a KAS factor B protein.

The above construct wherein said species is C. *hookeriana* or C. *pullcherrima.*

The above construct wherein said encoding sequence is chKAS B-2.

The above construct wherein said encoding sequence is chKAS B-31-7.

The above construct wherein said encoding sequence is chKAS A-2-7.

The above construct wherein said encoding sequence is chKAS A-1-6.

The above construct wherein said encoding sequence is cpuKAS B/7-8.

The above construct wherein said encoding sequence is cpuKAS B/8-7A.

The above construct wherein said encoding sequence is cpuKAS A/p8-9A.

The above construct wherein said encoding sequence is chKASIII-27.

An improved method for producing medium-chain fatty acids in transgenic plant seeds by expression of a plant medium-chain thioesterase protein heterologous to said transgenic plant,
the improvement comprising expression of a plant synthase factor protein heterologous to said transgenic plant in conjunction with expression of said plant medium-chain thioesterase, whereby the percentage of medium-chain fatty acids produced in seeds expressing both a plant synthase factor protein and a plant medium-chain thioesterase protein is increased as compared to the percentage of medium-chain fatty acids produced in seeds expressing only said plant medium-chain thioesterase protein.

The above method wherein said medium-chain thioesterase protein is a ChFatB2 protein.

The above method wherein said medium-chain thioesterase protein is a CpFatB1 protein.

The above method wherein said medium-chain thioesterase protein is a C12 preferring thioesterase from California bay.

The above method wherein said plant synthase factor protein is expressed from a construct as defined above.

The above method wherein said synthase factor A protein is from a *Cuphea* species.

The above method wherein said *Cuphea* species is *C. hookeriana* or *C. pullcherrima.*

A method of altering the medium-chain fatty acid composition in plant seeds expressing a heterologous plant medium-chain preferring thioesterase, wherein said method comprises
providing for expression of a plant synthase factor protein heterologous to said transgenic plant in conjunction with expression of a plant medium-chain thioesterase protein heterologous to said transgenic plant, whereby the composition of medium-chain fatty acids produced in said seeds is modified as compared to the composition of medium-chain fatty acids produced in seeds expressing said plant medium-chain thioesterase protein in the absence of expression of said plant synthase factor protein.

The above method wherein said medium-chain thioesterase protein is a ChFatB2 protein.

The above method wherein said medium-chain thioesterase protein is a CpFatB1 protein.

The above method wherein said medium-chain thioesterase protein is a C12 preferring thioesterase from California bay.

The above method wherein said plant synthase factor protein is expressed from a construct according to Claim 1.

The above method wherein said synthase factor A protein is from a *Cuphea* species.

The above method wherein said Cuphea species is C. *hookeriana* or C. *pullcherrima.*

The above method wherein said fatty acid composition is enriched for C10 fatty acids.

The above method wherein said fatty acid composition is enriched for C12 fatty acids.

The above method wherein said fatty acid composition is enriched for at least one medium chain fatty acid and at least one other medium chain fatty acid is decreased.

The above method wherein said enriched fatty acid is C12 and said decreased fatty acid is C14.

## Claims

1. A DNA construct comprising an encoding sequence for a ketoacyl-ACP synthase (KAS) protein selected from chKAS B-2 shown in Fig. 1, chKAS B-31-7 shown in Fig. 2, chKAS A-2-7 shown in Fig. 3, chKAS A-1-6 shown in Fig. 4, cpuKAS B/7-8 shown in Fig. 5, cpuKAS B/8-7A shown in Fig. 6, cpuKAS A/p7-6A shown in Fig. 7, cpuKAS A/p8-9A shown in Fig, 8 and chKAS III-27 shown in Fig. 9, preferably a chKAS A-2-7 shown in Fig. 3.

2. The DNA construct of claim 14 which comprises a coding sequence as shown in Fig. 1 to 9.
